# EUROPEAN PATENT SPECIFICATION

(11) **EP 3 606 662 B1**
(45) Date of publication and mention of the grant of the patent: **24.09.2025**
(21) Application number: 18719242.2
(22) Date of filing: 09.04.2018
(51) Int. Cl.: B01J 20/20, B01J 20/34, B01J 20/32, B01D 15/00, C01B 32/20, C01B 32/30, C02F 1/28

(54) **ADSORBENTS FOR TREATING CONTAMINATED LIQUIDS**
ADSORPTIONSMITTEL ZUR BEHANDLUNG VON KONTAMINIERTEN FLÜSSIGKEITEN
ADSORBANTS POUR LE TRAITEMENT DE LIQUIDES CONTAMINÉS

(30) Priority: 07.04.2017 GB 201705647
(43) Date of publication of application: 12.02.2020
(73) Proprietor: Arvia Technology Limited, Runcorn, Cheshire WA7 4EB (GB)
(72) Inventor: BROWN, Nigel, Runcorn Cheshire WA7 4EB (GB); KWAME, Nkrumah-Amoako, Runcorn Cheshire WA7 4EB (GB); ROBERTS, Edward, Runcorn Cheshire WA7 4EB (GB); HOLMES, Stuart, Runcorn Cheshire WA7 4EB (GB)
(74) Representative: Marks & Clerk LLP
(86) International application number: PCT/GB2018/050937
(87) International publication number: WO 2018/185501

(56) References cited:
- CN-A- 102 568 849
- GB-A- 2 495 701
- GB-A- 247 568
- GB-A- 883 021
- JP-A- H 038 440
- US-A- 4 081 370
- US-A- 5 215 690
- US-A1- 2003 003 289
- US-A1- 2003 042 205
- HUSSAIN S N ET AL: "Oxidation of phenol and the adsorption of breakdown products using a graphite adsorbent with electrochemical regeneration", ELECTROCHIMICA ACTA, ELSEVIER SCIENCE PUBLISHERS, BARKING, GB, vol. 92, 11 January 2013 (2013-01-11), pages 20 - 30, XP028984002, ISSN: 0013-4686, DOI: 10.1016/J.ELECTACTA.2013.01.020

## Description

The present invention relates to methods for producing adsorbent materials, in particular, materials for the treatment of contaminated liquids. It also relates to the products formed by the methods of the present invention. The adsorbent materials have particular, but not exclusive, application in the treatment of liquids to remove organic pollutants or contaminants.

Many methods have been developed to decontaminate liquids containing undesirable or unwanted species. Prior art methods typically exploit the process of absorption in which a contaminated liquid is contacted by a suitable absorbent material which has an affinity and capacity to absorb the contaminant from the bulk liquid phase into the pores of the absorbent material. Such a process is, however, only effective when the contaminant is present as a dispersed phase in the liquid. Absorption is not effective in the removal of dissolved contaminants from liquids.

As an alternative to absorption, the use of adsorbent materials to treat contaminated liquid has been investigated. Carbon-based adsorbent materials are particularly useful, and are capable of regeneration by the passage of an electric current through the adsorbent material. In use, a voltage is applied between electrodes, either continuously or intermittently, to pass current through the adsorbent material and regenerate it in the manner described in *"*Electrochemical regeneration of a carbon-based adsorbent loaded with crystal violet dye"; N W Brown, E P L Roberts, AA Garforth and R A W Dryfe; Electrachemica Acta 49 (2004) 3269-3281, and *"*Atrazine removal using adsorption and electrochemical regeneration"; N W Brown, E P L Roberts, A Chasiotis, T Cherdron, and N Sanghrajka; Water Research 39 (2004) 3067-3074. The use of carbon-based adsorbents in the treatment of contaminated water is also described in *"*Electrochemical pre-treatment of effluents containing chlorinated compounds using an adsorbent"; N. W. Brown and E.P.L Roberts, 2007, Journal of Applied Electrochemistry, Vol. 37, 1329-1335.

Suitable carbon-based adsorbents for use in the treatment of contaminated liquids are described in GB patent no. 2442950. This patent describes the use of unexpanded intercalated graphite in particulate form as an adsorbent particulate product for treating contaminated fluid, in which the unexpanded intercalated graphite is capable of electrochemical regeneration.

The materials produced by the method of the present invention are suitable for use in the treatment of contaminated liquids. Apparatus and a method for the treatment of a contaminated liquid to remove contaminants from said liquid are disclosed in GB patent no. 2495701. This patent describes the use of a bed of carbon-based adsorbent capable of electrochemical regeneration whereby contaminated liquid is contacted with the adsorbent material at a flow rate which is sufficiently high to pass the liquid through the bed, but below the flow rate required to fluidise the bed of adsorbent material. At least one pair of electrodes is provided to pass an electric current through the bed to regenerate the adsorbent material.

Apparatus and a method for the treatment of liquids using regeneratable adsorbent material is disclosed in GB patent no. 2475168. This patent describes a method for the treatment of a liquid comprising contacting the liquid within a treatment zone with an adsorbent material, passing the adsorbent material to a regeneration zone within the treatment zone after contact with said liquid and electrochemically regenerating the adsorbent material within the regeneration zone, a disinfectant precursor species being provided in the regeneration zone, subjected to electrochemical conversion to generate a disinfectant species within the regeneration zone and adsorbent material and/or liquid within the regeneration zone contacted by said disinfectant species, wherein regeneration of the adsorbent material is effected simultaneously with oxidation of the disinfectant precursor species.

GB patent no. 2470042 discloses a method and apparatus for removing contaminants from a quantity of contaminated liquid. The method comprises delivering the contaminated liquid to a treatment reservoir containing carbon-based material capable of electrochemical regeneration in the form of a bed of particles at the base of the reservoir, agitating the bed to distribute the adsorbent material in the liquid and adsorb contaminant therefrom, ceasing the agitation and allowing the bed of material to settle, regenerating the adsorbent by passing an electric current through the bed to release from the adsorbent gaseous products derived from the contaminant in bubbles rising through the liquid in the reservoir, and removing the decontaminated liquid from the tank. All of the above are referred to generically as the Arvia^{™} Process.

US 4081370 A discloses carbon particulates comprising carbon black spheres and a carbon binder having large pores as well as desirable pore size distributions that are useful as selective adsorbents.

The adsorbent materials of the prior art used in the Arvia^{™} Process have a relatively low surface area of around 1.2 m² g⁻¹, which imparts low adsorption capacity. Adsorption isotherms show a saturated adsorbate loading of between 1 and 3 mg g⁻¹ for a variety of organic contaminants treated. In addition, the continuous regeneration of the adsorbent materials of the prior art can lead to attrition of the material thereby reducing its capacity to treat the contaminated liquid and possibly even adding to the turbidity of the treated liquid.

Whilst the materials of the prior art display all of the properties required to be used in the commercial treatment of contaminated liquid, it is desirable to provide a material which has improved properties over those known in the prior art.

There is therefore a need for improved adsorbent materials, which can be used to adsorb pollutants from contaminated liquids and which can be electrochemically regenerated. There is also a need to have adsorbent materials with improved adsorptive and/or conductive properties that are resistant to attrition.

In the methods of the prior art, a monolith block comprising a single type of particle is produced, which may then be milled to produce particles. However, this results in a wide particle-size distribution, which is undesirable. In the Arvia^{™} process, the adsorbent particles are allowed to settle into a bed. If there is a large range of particle sizes, the bed formed might not be homogenous and this could have a negative impact on the regeneration of the adsorbent bed. In most cases, effluent is passed through a packed bed in downflow mode, i.e. where the flow of effluent is generally from the top of the bed down through to the bottom of the bed. However, in certain cases, the effluent can be passed through the bed in upflow mode, i.e. where the flow of effluent is generally from the bottom of the bed to the top of the bed. A bed which is homogenous allows all of the bed to be used. Similarly, a more uniform size of particle results in improved packing of the bed, which improves bed conductivity and therefore electrochemical regeneration.

An object of the present invention is to obviate or mitigate the problems associated with existing adsorbent materials for treating contaminated liquids and to provide for alternative methods for producing adsorbent materials.

According to a first aspect of the present invention, there is provided a method of making a particulate adsorbent material for the treatment of contaminated liquids comprising mixing a first particulate material with a second material, wherein both the first and second materials are particulate, the first and second materials are different, the first material is electrically conductive and the second material is adsorptive, homogenising the mixture of the first and second materials, incorporating an impregnating or coating material capable of carbonisation, and carbonising the mixture, the first material is powdered graphite and the second material is powdered activated carbon.

The capability of materials to undergo electrochemical regeneration will depend upon their electrical conductivity, surface chemistry, electrochemical activity, morphology, electrochemical corrosion characteristics, and the complex interaction of these factors. A degree of electrical conductivity is necessary for electrochemical regeneration and a high electrical conductivity can be advantageous. Additionally, the kinetics of the electrochemical oxidation of the adsorbate is desirably fast. The kinetics depends upon the electrochemical activity of the adsorbent surface for the oxidation reactions which occur when the adsorbate is destroyed. Additionally, electrochemical regeneration will generate very corrosive conditions at the adsorbent surface. The electrochemical corrosion rate of the adsorbent material under regeneration conditions should be low so that the adsorption performance does not deteriorate during repeated cycles of adsorption and regeneration. Additionally, some materials can passivate upon attempted electrochemical regeneration, often due to the formation of a surface layer of non-conducting material. This may occur, for example, as a result of the polymerisation of the contaminant, for example phenol, on the surface of the adsorbent. In addition, electrochemical destruction of the contaminants on the adsorbent material will generate reaction products which must be transported away from the surface of the adsorbent material. The ability for the adsorbent material being regenerated to successfully transport the products away from the surface of the adsorbent material will depend upon both the surface structure and chemistry of the adsorbent material.

It will be appreciated that the materials produced by the method of the present invention will have the ability to adsorb. The process of adsorption works by a molecular interaction between the contaminant and the surface of the adsorbent. In contrast, the process of absorption involves the collection and at least temporary retention of a contaminant within the bulk of the material.

By way of example, expanded graphite is known to be a good absorber of a range of contaminants (e.g. up to 86 grams of oil can be "taken-up" per gram of compound). However, there is generally low absorption or uptake of dissolved compounds. Unexpanded graphite intercalation compounds have effectively no absorption capacity. They can adsorb dissolved organics, but the adsorption capacity is generally very low as the surface area is low (e.g. up to 7 milligrams of oil can be "taken-up" per gram of compound). The adsorption of non-soluble compounds may result in a coating of the adsorbent particle. These figures demonstrate a difference of four orders of magnitude between the take-up capacity of expanded graphite and that of unexpanded graphite intercalation compounds. However, the selection of which materials to select is a careful balance of the take-up capacity of the material versus the ease and efficiency by which the material can be regenerated.

The method of the first aspect of the present invention makes it possible to control the properties of the adsorbent material by selecting the first and second materials. The properties which may be of interest to be controlled include adsorptive capacity, conductivity, resistance to attrition, density, and electrochemical regenerative capacity. Controlling these properties allows the adsorbent material to be tailored to the particular task for which it is to be used. For example, the first particulate material is chosen for its electrical conductivity, whilst the second material is chosen for its adsorptive properties. As such, in circumstances where it is desirable to have a high current pass through the adsorbent material, it may be advantageous to select materials which result in an adsorbent material having good conductivity (low resistance) and it is possible to sacrifice some adsorptive capacity. Alternatively, in high attrition applications, it may be desirable to choose materials which result in an adsorbent material which is resistant to attrition, but which have lower adsorptive capacity and/or conductivity. The method of the present invention allows a uniform size of adsorbent particle to be produced. Having particles of uniform size means that the settling rate of the particles is also uniform. Where particles are of a non-uniform size, they will settle out of solution at different rates and may form beds having layers comprising differently sized particles. In cases where an electric potential is applied across the bed, this may result in different currents passing through the layers of differently sized particles, which may be undesirable. Therefore, having a uniform particle size allows a homogenous bed to be formed.

At least one of the first and second materials is substantially non-porous. Both of the first and second materials may be substantially non-porous.

The impregnating or coating material may be curable. By curable, it is understood that the physical properties of the material may be altered by chemical or physical means. In particular, a curable material is one which is able to be hardened by chemical or physical means. For example, polymerisation of a monomer is one type of curing. Thus, a monomer is a curable material. Curing is essentially an irreversible process. Where the impregnating material is curable, the method may include curing the mixture. The curing may take place prior to or simultaneously with carbonisation.

Alternatively, the impregnating or coating material may be non-curable. In the context of the present application, a non-curable material is one which is not irreversibly altered by chemical or physical means. For example, a thermoplastic is a non-curable material since it may be reversibly changed between a soft state when heated and a hard state when cooled. It will be appreciated that the various features of the first aspect of the present invention may be used in combination with methods in which the impregnating or coating material is curable as well as in methods in which the impregnating or coating material is non-curable.

The second material and the impregnating or coating material are separate materials.

Both of the first and second materials are particulate.

The first and second materials are different.

The incorporation of the impregnating or coating material capable of carbonisation may be carried out before, during, or after the first and second materials are mixed. As such, one or both of the first and second materials may be mixed with the impregnating or coating material prior to the first and second materials being mixed together.

The first and second materials may be mixed in any ratio. Preferably, the first and second materials are mixed in ratios from around 1:99 to around 99:1, from around 10:90 to around 90:10, from around 20:80 to around 80:20, from around 30:70 to around 70:30, from around 40:60 to around 60:40, and around 50:50, all by weight.

One or more further materials may be present in addition to the first and second materials.

The impregnating or coating material may be a polymerisable organic compound. The impregnating or coating material may be a thermosetting resin, a thermoplastic, or a polymerisable compound, such as a monomer. The impregnating or coating material may comprise a mixture of one or more thermosetting resins or thermoplastics or polymerisable compounds. An impregnating material may be absorbed into the bulk of at least one of the first and second materials and a may also coat at least a portion of the surface of the materials. A coating material may coat at least a portion of the surface of the materials and may or may not be absorbed into the bulk of at least one of the first and second materials.

Where the impregnating or coating material is a thermoplastic, no curing may be required.

Where the impregnating or coating material is a thermosetting resin or polymerisable compound, it may be necessary to cure the resin or polymerisable compound. The curing may be achieved through any appropriate method. Preferably, the curing is achieved by thermal treatment, use of a chemical initiator, or a combination of the two. The chemical initiator may be an acid or an alkali. Preferably, the chemical initiator is an acid. Preferably, curing results from the polymerisation of the impregnating or coating material.

The impregnating or coating material may be selected from the list comprising phenolic resins, furan resins, oxidised polystyrene, polyvinyl alcohol, polyacrylonitrile, polyvinylidene chloride, cellulose, epoxy resins, polystyrene, sucrose, and polymethylmethacrylate. Preferably, the impregnating or coating material is furfuryl alcohol. The purpose of the impregnating or coating material is to bind the first and the second materials together following carbonisation or to provide a source of carbon to coat the first material. Without wishing to be bound by scientific theory, it is believed that the impregnating or coating material provides a source of carbon which binds the particles of the first and second materials together following carbonisation, or provides a carbon source which coats the first material when the second material and the impregnating or coating material are the same.

The first and second materials may be impregnated or coated by the impregnating or coating material by any suitable means. The impregnation or coating may be effected by soaking the first and second materials in the impregnating or coating material. Where the second material is the impregnating or coating material, the first material is soaked in the second material. The mixture may be mechanically stirred to ensure homogeneity of the mixture. Optionally or additionally, the soaked materials may be subjected to vacuum-pressure cycles. In a vacuum-pressure cycle, the mixture is placed under vacuum for a period of time after which it is brought back to atmospheric pressure. This may be repeated as often as required. The vacuum and the length of time for which the mixture is placed under vacuum may be chosen from any suitable pressure and time. The vacuum may be around 30 mm Hg above absolute pressure. The material may be held under vacuum for around 3 hours. The material may be held at atmospheric pressure for around thirty minutes. However, the skilled person would appreciate that the pressures used in the vacuum and the times for which the material is held under vacuum/at atmospheric pressure may be varied in accordance with common practice.

For example, the mixture of the first and second materials may be mixed with furfuryl alcohol and allowed to soak in ambient conditions in excess 0.5 M HCl. The mixture may be subject to vacuum-pressure cycles. The mixture may be allowed to dry in ambient conditions. The mixture may then be heated to a temperature sufficient to polymerise the furfuryl alcohol and held at the temperature for a period of time sufficient to allow substantially all of the furfuryl alcohol to polymerise. The material may be carbonised.

The method may include the addition of water in addition to the impregnating or coating material. Without wishing to be bound by scientific theory, it is believed that the addition of water facilitates the homogenisation of the mixture.

In the method of the first aspect of the present invention, the mixture may be dried before the material is carbonised.

In the method of the first aspect of the present invention, the mixture may be washed where appropriate.

In the method of the first aspect of the present invention, following carbonisation, the resulting material may be activated. The material may be crushed following carbonisation or following activation. Preferably, washing takes place after crushing and/or activation.

Homogenising the mixture of the first and second materials and incorporating an impregnating or coating material capable of carbonisation may be carried out in any order. For example, the mixture of the first and second materials may be homogenised prior to the incorporation of the impregnating or coating material capable of carbonisation, or the impregnating or coating material capable of carbonisation may be incorporated into the mixture prior to homogenisation. Alternatively, the homogenisation and the incorporation of the impregnating or coating material capable of carbonisation may take place simultaneously.

Carbonisation is the conversion of the impregnating or coating material into carbon. Without wishing to be bound by scientific theory, it is believed that it is the pyrolysis process that forms a cross-linked and porous carbon matrix of the first and second materials in the composite material of the present invention.

The carbonisation may be carried out under any suitable conditions. In one example, the material may be heated from ambient temperature to around 550°C at an initial ramp rate of 5°C min⁻¹ and held at that temperature for around one hour. The material may then be heated at the same ramp rate to around 700°C and held at this temperature for around an hour. The material may then be allowed to cool to ambient temperature by convection. The carbonisation may be carried out in a flow of nitrogen. The skilled person would understand that any heating profile could be used which results in the carbonisation of the impregnating material.

The carbonised material may be crushed by any suitable means.

The carbonised material may be activated. Without wishing to be bound by scientific theory, it is believed that activation of the materials of the present invention increases the adsorption capacity of the materials by increasing the number of 'active sites' on the surface of the material for adsorption. The activation may be effected by physical or chemical means.

Physical activation may be effected by heating the carbonised material up to around 800°C to around 900°C. The materials may then be purged with one or a combination of gases suitable for providing an oxidative atmosphere. Suitable gases include steam, carbon dioxide and carbon monoxide. Without wishing to be bound by scientific theory, it is believed that the gases provide an oxidative atmosphere which initiates the oxidation of the carbonised material into an activated material.

Chemical activation may be effected by the use of chemicals such as acids, salts or bases. Examples of suitable chemicals include phosphoric acid, zinc chloride, potassium hydroxide, and sodium hydroxide.

Without wishing to be bound by scientific theory, it is believed that the mechanism of activation for carbonised materials by a strong base, such as potassium hydroxide, results from a fusion between the strong base pellets and the carbonised material which leads to intercalation of the cations from the strong base, for example potassium ions, into the structure of the carbonised material. At around 300°C, potassium hydroxide dehydrates to form potassium oxide (K₂O). This dehydration causes expansion and this separates the layers of the carbonised material. Further, at around 700°C, the K₂O is reduced by the carbon in the carbonised material. It is believed that this consumption of carbon for the reduction of the K₂O that results in the development of pores.

The activation of the carbonised material may be achieved by soaking the carbonised material in an aqueous solution of potassium hydroxide. The material may be soaked in the KOH solution overnight under ambient conditions and optionally the material may be subject to vacuum-pressure cycles. The material may then be dried and held at a temperature of around 120°C to evaporate the water. The dried carbonised material may then be activated by heating the material to around 700°C and holding it at that temperature for a time sufficient to allow the material to be activated. The skilled person would be able to readily determine the temperature and times required to allow the material to be activated.

Preferably, the first and the second materials are bound to the surface of one another. In a preferred embodiment, the first or second materials are not bound within a pore of the other of the first or second material.

It is desirable that the method of the present invention does not involve compression of the materials. It is also desirable that the materials formed by the method of the present invention are not compressed. Preferably, the material produced by the method of the present invention is not a monolith.

According to a second aspect of the present invention, there is provided an adsorbent material which is made by, or is obtainable by, the process of the first aspect of the present invention. There is also provided the use of an adsorbent material which is made by, or is obtainable by, the process of the first aspect of the present invention in the treatment of a contaminated liquid.

As described above, the adsorbent material produced by the method of the first aspect of the present invention may have its physical and chemical properties attuned to the particular task for which it is to be used. This is advantageous as it allows the most efficient use of the adsorbent material for a given purpose. Since adsorbent materials may be used in a wide number of industries, including waste water treatment and nuclear processing, the requirements for an adsorbent material capable of electrochemical regeneration are diverse. For example, the nuclear industry generally requires high charge applications and so it is desirable to have an adsorbent material with high conductivity. On the other hand, where the adsorbent material is to be used to adsorb and electrochemically destroy micro-pollutants, the adsorptive capacity may be more important, whether to specifically adsorb a particular micro-pollutant or to absorb an increased amount of the micro-pollutant.

According to a third aspect of the present invention, there is provided a method of treating the surface of an adsorbent material comprising passing a current through the adsorbent material in the absence of adsorbed contaminants, wherein the adsorbent material is made by, or obtainable by, the first aspect of the present invention. The method of passing a current through an adsorbent material in the absence of adsorbed contaminants may be used to alter the surface properties of the adsorbent, which can be achieved either with or without the presence of an electrolyte. There is also provided a material made by, or obtainable by, the method according to the third aspect of the present invention.

According to a fourth aspect of the present invention, there is provided a method of removing contaminants from a quantity of contaminated liquid comprising passing an electric current through an adsorbent material prior to contacting it with the contaminated liquid, contacting the adsorbent with the contaminated liquid, allowing the adsorbent material to adsorb contaminants from the contaminated liquid, and regenerating the adsorbent by passing an electric current through the adsorbent, wherein the adsorbent material is made by, or obtainable by, the first aspect of the present invention.

The treatment and regeneration process can be continuous, semi-continuous or batch. An individual volume of liquid can be treated as a batch, with the adsorbent material being regenerated prior to the liquid being introduced, and then regenerated as the respective batch is treated or between batch treatments. Regeneration is effected by passing a current through the adsorbent. Some compounds may also be treated within an undivided cell, provided there is no continuous electrical connection between the cathode and anode through the solid conducting adsorbent material. In a continuous or semi-continuous process, the flow rate of the liquid through the apparatus is determined and controlled to ensure a sufficient dwell time in contact with the recycling adsorbent.

In conventional systems relying upon adsorption followed by electrochemical regeneration of the adsorbent, the contaminant is allowed to adsorb onto the adsorbent and a current is passed through the adsorbent in order to regenerate said adsorbent. The passage of current through an adsorbent material in the absence of adsorbed contaminants therefore amounts to pre-regeneration of the adsorbent and does not serve to break down any contaminants since none are present. However, it has been surprisingly discovered that pre-regeneration of an adsorbent serves to increase the adsorptive capacity of the adsorbent and consequently the adsorbent is able to adsorb more contaminants when it is mixed with a contaminated liquid when compared to an adsorbent which has not been pre-treated. This can result in efficiency gains and can increase the amount of contaminants adsorbed and removed from a contaminated liquid by the adsorbent.

The methods according to the third and fourth aspects of the present invention can be used in connection with the apparatuses and methods disclosed in any of Arvia's earlier patents and patent applications, such as WO2007/125334 and WO2010/128298 as well as those disclosed in the application entitled "Apparatus and Methods for Aqueous Organic Waste Treatment" filed on the same date as the present application by Arvia Technology Limited. In particular, the adsorbent may be regenerated by passing a current through it prior to the adsorbent adsorbing any contaminants. The adsorbent materials according to any aspect of the present invention may be used as the adsorbent material in any aspect of the application entitled "Apparatus and Methods for Aqueous Organic Waste Treatment" filed on the same date as the present application by Arvia Technology Limited.

Furthermore, in addition to increasing the current applied to the bed of adsorbent material in order to treat organic contaminants with a high oxidation potential, it has also been surprisingly realised that it is possible to boost the oxidation potential in a system by using a chemical additive. In particular, it has been surprisingly realised that the addition of hydrogen peroxide can enhance the performance of the system.

When added to a water treatment apparatus, the hydrogen peroxide is reduced at the cathode to form water and a hydroxyl radical. The hydrogen peroxide will also increase the generation of hydroxyl radicals in the anodic zone, with treatment generally occurring in the anodic bed, however the addition of hydrogen peroxide results in the production of a strong oxidising agent in the cathodic bed. As such, oxidation can be achieved in both the anodic and cathodic beds.

The oxidation potential of the hydroxyl radical produced is 2.8 V, which is greater than that of ozone (2.08 V), chlorine (1.36 V) or hydrogen peroxide (1.78 V).

As such, there is provided the use of hydrogen peroxide in the apparatus and methods of any aspect of the present invention. The concentration of hydrogen peroxide may be maintained by addition of further amounts of hydrogen peroxide to balance the rate at which the hydrogen peroxide is consumed.

In the apparatus and methods of the prior art and the co-pending application entitled "Apparatus and Methods for Aqueous Organic Waste Treatment" outlined above, the electric current feeders may be operated continuously. In other embodiments, the electric current feeders may be operated intermittently.

In systems of the prior art, the key feature is that adsorption and electrochemical destruction of adsorbed contaminants takes place simultaneously, which allows for continuous treatment. However, some contaminants require relatively high voltages to achieve oxidation. For example, metaldehyde requires a minimum cell potential of 3 volts to ensure that the oxidation potential at the adsorbent surface is high enough to achieve organic oxidation. The higher oxidation potential can be achieved by increasing the current density, but this would result in an increase in power through both increased current and voltage, which results in higher costs.

Where there are only low concentrations of organic contaminants requiring high oxidation potentials in the liquid to be treated, only a small charge, but high voltage, may be required to oxidise the contaminants. If the current is applied continuously, only a small percentage of the charge is used to oxidise the contaminants and the rest is wasted on side reactions. This results in low current efficiencies. In addition, the increased oxidation potential and the large number of excess electrons can result in oxidative damage to the adsorbent material itself.

It has been surprisingly realised that it is possible to operate such apparatus and methods in an alternative manner by making use of the adsorptive capacity of the adsorbent material. The liquid to be treated may be passed through the bed of adsorbent material continuously resulting in the contaminants in the liquid being continuously adsorbed and concentrated on the surface of the adsorbent material. Due to the adsorptive capacity of the adsorbent material, the liquid may be passed through the bed of adsorbent material for some time before organic breakthrough occurs. Before organic breakthrough occurs, the current may be turned on at a current density high enough to produce the voltage required for oxidation of the particular compounds to be treated, in particular the organic materials which are adsorbed onto the surface of the adsorbent material. When the current is being applied, oxidation of the contaminants takes place and thereby regenerates the surface of the adsorbent to allow further contaminants to be adsorbed. The period of applying the current may be less than the period required for adsorption. Since the current is only applied intermittently, although the same current density is required, it is required for a shorter period of time. As such, the energy requirements are lower overall and cost savings can be achieved. In addition, the damage to adsorbent material through side reactions may also be reduced. It should also be appreciated that during the intermittent regeneration, adsorption can still continue.

Although the intermittent application of current to regenerate the beds of adsorbent material has particular application in respect of the present invention, the skilled person would recognise that methods and apparatus for treating contaminated liquids utilising adsorbent materials may also benefit from intermittent operation of the current feeders.

As such, the current feeders may be operated intermittently. Preferably, the current feeders are operated prior to organic breakthrough occurs. The adsorbent materials and methods according to any aspect of the present invention may be used in a system in which there is intermittent application of current and therefore intermittent regeneration of the adsorbent material.

The current feeders may be operated at a first voltage which is sufficiently high to result in oxidation of a first contaminant and intermittently operated at a second voltage which is higher than the first voltage in order to oxidise a second contaminant. As such, the current can be varied to intermittently oxidise organic contaminants in the liquid to be treated. The current may be completely turned off between periods when the current is increased to a level required to oxidise adsorbed contaminants, or it may be reduced to a lower level in order to maintain a degree of current passing through the adsorbent material.

The variation in current densities applied to the adsorbent materials may be advantageous in cases where there is more than one contaminant in the liquid, the contaminants may require different oxidation potentials to be oxidised. In the prior art, the current density would have been held at a level required to oxidise the contaminant with the highest oxidation potential. As such, the power requirement would be high and energy costs would also be high.

It is possible to use the adsorbent materials of the present invention in a solar powered water treatment system. In such systems, the current feeders may be connected to a photovoltaic cell, commonly referred to as a solar panel. During the day, the solar panel is able to generate direct current which can be passed to the current feeders and used to effect electrochemical oxidation of adsorbed contaminants. The power generated by the solar panel will vary during the day and will peak when the sun is at its strongest. Thus, adsorbed contaminants may be treated during the day. Following treatment, the treated liquid may be taken off and replaced with untreated liquid. The contaminants in the untreated liquid may be allowed to adsorb to the adsorbent material overnight and then be destroyed the next day when solar power is available once more. The ability to tune the physical and electrochemical properties of the adsorbent materials of the present invention using the methods of the present invention allow the adsorbent material to be tailored to allow the power produced by solar panels to regenerate the adsorbent material.

The methods of the present application allow for the size of the particles of adsorbent materials to be altered depending on the application to which the particles are intended to be used. For example, in systems where an upflow of contaminated liquid is used, it may be desirable to use larger particles. In particular, where a high flow rate is required, a downward flow may compact the bed of adsorbent material. The use of larger particles allows an improved flow rate, but at the cost of a higher potential difference required and thus a higher power requirement. However, this may be offset by the ability to use a higher flow rate.

Similarly, the methods of the present invention allow the production of adsorbent materials which have the desired physical characteristics for a particular application. The methods of the present invention allow for adsorbent materials to be produced for a wide range of applications. It is possible to fine-tune the physical characteristics of the final adsorbent materials, such as the size, conductivity or adsorptive capacity or specificity, by altering the nature of the first and/or second materials used. Therefore, the methods of the present invention allow for the adsorptive and conductive properties of an adsorbent to be controlled to meet particular requirements.

The methods of the present invention allow adsorbent materials having different sizes to be produced and tailored for particular applications. Using the methods of the present invention, it is possible to carefully control the size of the adsorbent materials produced. Different applications will require different sizes of particles. For example, small particles of around 2 to 5 mm in length is the typical particle size for removal or organic compounds where there is a need for higher surface areas to increase the quantity of organic contaminant removed and may be used for the treatment of industrial wastewaters. Particles in the size range of around 5 to 10 mm are used where there is a need for both organic compound and microorganism treatment, but where the load of both is not high. Particles in the size range of 10 to 15 mm are useful in applications requiring the removal or bacteria and/or other microorganisms, such as drinking water applications or water recycle/reuse systems. Finally, particles in the size range of around 15 to 20 mm are useful for high flow applications where there is a very low organic load and are particularly useful in systems where the water is recycled or recirculated, for example cleaning water in aquaria and fountains.

The disclosure will now be described by way of example and with reference to the accompanying figures wherein:
Figure 1 is a graph of the relationship between the voltage and bed depth at a series of different currents and used to calculate the conductivity of the beds;
Figures 2a and 2b are scanning electron microscope (SEM) images of Nyex^{™} and Nyex^{™} impregnated with furfuryl alcohol;
Figure 3 is a graph of the concentration of AV-17 over time used to estimate the adsorption equilibrium of a composite material comprising compressed expanded graphite (GEC) and granulated activated carbon (GAC);
Figures 4a and 4b show the isotherm of adsorption of AV-17 onto non-activated CEG-GAC composite adsorbent and the isotherm of adsorption of AV-17 onto activated CEG-GAC composite adsorbent respectively;
Figure 5 shows the regeneration efficiencies of the CEG-GAC composite materials for the adsorption of AV-17 over four adsorption-regeneration cycles;
Figure 6 is graph of the adsorption of AV-17 from solution by a composite material comprising graphite and activated carbon;
Figures 7a and 7b show the dynamic light scattering measurement showing the particle size distribution of powdered graphite and the particle size distribution of powdered activated carbon respectively;
Figures 8a, 8b, and 8c show the kinetic results of the uptake of resorcinol by PGPAC6040 (a composite material comprising around 60% by weight powdered graphite (PG) and about 40% by weight powdered activated carbon (PAC)), PGPAC5050 (a composite material comprising PG and PAC is approximately equal proportions by weight), and PGPAC4060 (a composite material comprising about 40% by weight PG and about 60% by weight PAC);
Figures 9a, 9b, and 9b show the kinetic results of the uptake of AV-17 by PGPAC6040, PGPAC5050, and PGPAC4060;
Figure 10 shows the adsorption efficiencies of the PGPAC variants over five cycles;
Figure 11 shows the turbidity over time of samples of water containing samples of NYEX^{™}, PGPAC4060, PGPAC5050, and PGPAC6040 in which the samples were stirred at 600 rpm;
Figure 12 shows the turbidity over time of samples of water containing samples of NYEX^{™}, PGPAC4060, PGPAC5050, and PGPAC6040 in which the samples were stirred at 800 rpm
Figure 13 shows the turbidity over time of samples of water containing samples of NYEX^{™}, PGPAC4060, PGPAC5050, and PGPAC6040 in which compressed air at 2 barg at a flow rate of 2 litres per minute was passed through the water; and
Figures 14a, 14b, 14c, and 14d show the turbidity of samples of water for four adsorption/regeneration cycles.

### Reference Example 1 - Prior art material - NYEX^{™}

In order to provide a reference against which the materials produced by the method of the present invention could be compared, the physical characteristics of the adsorbent NYEX^{™} were determined. Details of the nature of NYEX^{™} may be found in GB2442950. NYEX^{™} is produced by Arvia Technology Limited.

The surface area of the NYEX^{™} was measure by nitrogen adsorption to be 1.2 m²g⁻¹. As will be appreciated, this is considerably less than the surface area for most activated carbon adsorbents, which have surface areas of in excess of 1200 m²g⁻¹.

The NYEX^{™} was measured to have no internal porosity, with the bulk and particulate densities measured as 0.5 to 1.0 g cm⁻³, and around 1.9 to 2.2 g cm⁻³ respectively, depending on particle size, particle shape and size distribution. The free particle settling velocity and hindered particle settling velocity were estimated to be 312 cm min⁻¹ and 102 cm min⁻¹ respectively. These values were verified experimentally as 267 cm min⁻¹ and 89 cm min⁻¹, which were comparable to over 85% confidence with each other.

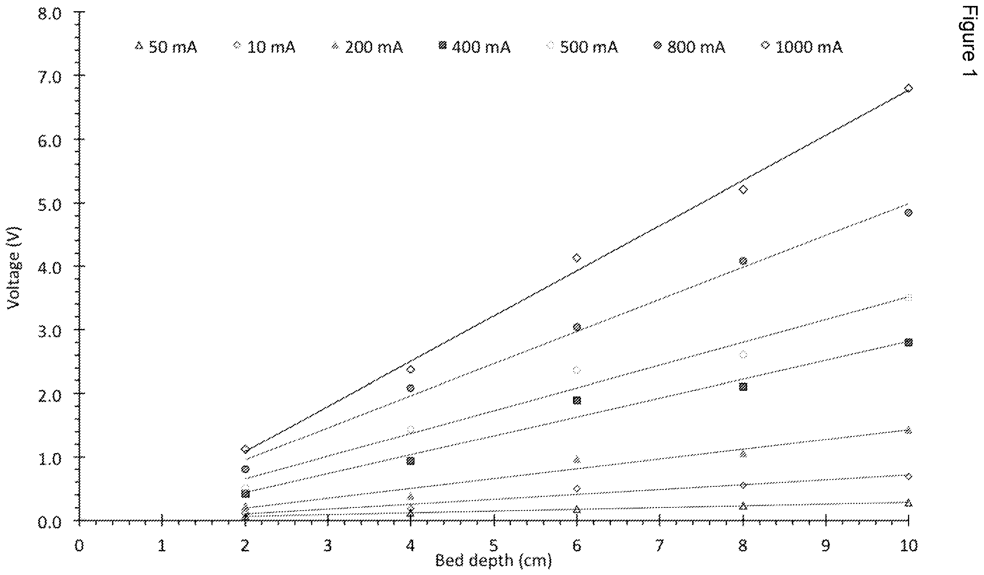

The electrical conductivity of a bed of NYEX^{™} was measured to be 0.24 ± 0.03 Ω⁻¹ cm⁻¹. The voltage versus depth plots and the conductivity data used to calculate the bed conductivity are shown in Figure 1 and Table 1 respectively.

**Table 1**

| Current (mA) | Potential drop (V cm⁻¹) | Resistivity (Ω cm) | Conductivity (Ω⁻¹ cm⁻¹) |
|---|---|---|---|
| 50 | 0.0282 | 3.4728 | 0.2879 |
| 100 | 0.0729 | 4.4888 | 0.2228 |
| 200 | 0.1461 | 4.4981 | 0.2223 |
| 400 | 0.2863 | 4.4072 | 0.2269 |
| 500 | 0.3537 | 4.3558 | 0.2296 |
| 800 | 0.5006 | 3.8531 | 0.2595 |
| 1000 | 0.7298 | 4.4938 | 0.2225 |

Granular activated carbon was measured to have a bed electrical conductivity of 0.08 ± 0.01 Ω⁻¹ cm⁻¹. As a further comparison, the conductors copper and stainless steel have electrical conductivities of 6 x 10⁶ Ω⁻¹ cm⁻¹ and 1.5 x 10⁶ Ω⁻¹ cm⁻¹ respectively, whereas de-ionised water and sea water have electrical conductivities of 5.5 x 10⁻⁶ Ω⁻¹ cm⁻¹ and 4.8 x 10⁻² Ω⁻¹ cm-¹ respectively. As can be seen from these relative values, NYEX^{™} is a relatively good conductor of electricity which means it may be used in electrochemical regeneration.

The adsorption kinetics of NYEX^{™} were investigated in respect of resorcinol and the dye acid violet-17 (AV17) by fluidising 50g of NYEX^{™} in a litre of solution of resorcinol or AV17 and measuring the concentration of the respective adsorbent. It was found that adsorption equilibrium was reached at around 45 minutes. As such, a time of 45 minutes was used for the adsorption experiments with NYEX^{™} as this had been shown to be long enough to achieve adsorption equilibrium.

Kinetic modelling of NYEX^{™} has indicated that adsorption is best described by a pseudo-second order model which is based on a combination of physical and chemisorption between the adsorbates and the adsorbent. This is considered likely as NYEX^{™} has been measured to be non-porous by a combination of nitrogen adsorption and mercury porosimetry, and has also been shown to contain surface functionalities which affect the rate of adsorption onto the material. In addition, adsorption isotherms of NYEX^{™} show that the adsorption is monolayer adsorption on a non-porous adsorbent.

The regeneration efficiency of NYEX^{™} was also measured over four adsorption and electrochemical regeneration cycles. The results indicated that NYEX^{™} maintained high regeneration efficiencies over the cycles.

Adsorbents are usually exposed to extreme chemical, mechanical and thermal forces. Material disintegration is one of the main contributing factors to material losses in adsorption systems. For example, in steam regeneration processes for activated carbon adsorption systems, material loss of about 15% is directly linked to the elevated pressures used in the process. It is believed that NYEX^{™} undergoes material degradation and attrition due to the mechanical action of the air injected into the adsorption system which provides agitation and mixing. This creates fines which may reduce the quality of the treated water and limits the adsorption capacity. Turbidity measurements were taken and are discussed in more detail below. However, it was found that NYEX^{™} does undergo a degree of material attrition when in continuous use as an adsorbent.

### Reference Example 2 - Compressed Expanded Graphite

This reference example relates to a method of increasing the surface area of NYEX^{™} to improve the surface area and thereby increase the adsorption capacity.

In consideration of the relatively low surface area of NYEX^{™}, it was investigated whether the adsorption capacity could be increased by making some of the internal surface available. This was investigated by exfoliating the NYEX^{™} which involves heating the material at a high temperature for a short time, for example 800°C for one minute. The volatisation of the intercalated species can result in a three hundred-fold expansion in the volume of the material.

To synthesise a material of this nature, a sample of NYEX^{™} was sieved to homogenise the particle size. NYEX^{™} has a particle size of around 500 microns, but there is dust present with a size of less than or equal to 140 microns. As such, the sample of NYEX^{™} was size classified using a sieve tower to remove the dust. Particle sizes in the range of 140 to 425 microns were chosen as this represented the largest size range, which ensured homogenous exfoliation.

5 g of the material were spread out into a stainless steel furnace tray and put into a muffle furnace preheated to 800°C for one minute. The tray was left to cool in ambient conditions.

A comparison of the regular and exfoliated NYEX^{™} showed a significant particle size increase. The expanded graphite particles were compressed into compressed expanded graphite (CEG) particles with a similar morphology as the original unexpanded particles, but with less density due to the increased porosity. Dynamic Light Scattering (DLS) was used to measure the particle size of the expanded material, which found a mean particle size of 739 microns.

The surface area of the CEG particles was measured by nitrogen adsorption to be 22.5 m² g⁻¹, which is a significant increase over the surface area of the NYEX^{™} material. The surface area of the expanded graphite before compression was measured to be 149 m² g⁻¹. The bulk density of the CEG materials was measured as 0.19 g cm⁻³ (compared to 0.53 g cm⁻³ for NYEX^{™}) and the particulate density was measured using helium pycnometry as 0.12 g cm⁻³ (compared to 1.91 g cm⁻³) for NYEX^{™}. The CEG particles were measured by nitrogen adsorption to contain mesopores and macropores.

The low density of the material may make it unsuitable for use as an adsorbent in liquid systems, including the Arvia^{™} system for the removal of contaminants from a liquid as described in GB2495701. In particular, since the GEC particles float, there may be inefficient contact between the adsorbent and the adsorbates, and there is significant loss of adsorbent due to overflow of the treatment tanks. In addition, poor settling velocity (which may in fact be negative since the material floats on water) makes regeneration of the adsorbent difficult.

The average dry bed electrical conductivity of the CEG adsorbent was measured as 0.17 ± 0.01 Ω⁻¹ cm⁻¹. This is less than the conductivity of the NYEX^{™} material, but still sufficient to allow electrochemical regeneration.

Since the CEG adsorbent floats on water, it proved difficult to conduct adsorption kinetics studies. However, adsorption equilibrium was believed to have been reached after around 60 minutes. Adsorption isotherm studies for the removal of AV-17 were carried out and the results show that the CEG material had a saturation adsorption capacity of about 7 mg g⁻¹, which is around double that of NYEX^{™}.

The regeneration efficiency of the CEG material was measured, but it was necessary to drain most of the liquid from the test rig due to the tendency of the CEG particles to float. The cell potentials for the CEG particles was measured to be more than twice the required cell potentials of NYEX ^{™} to supply a current of 1 Amp required for regeneration. As such, the electrical energy cost for regeneration of the CEG particles is double that of NYEX^{™}. The average regeneration capacity of the CEG particles was calculated to be 97% across four adsorption/regeneration cycles.

As such, although the CEG material offered improved surface area over NYEX^{™} and double the adsorptive capacity, the low density of the particles meant that they did not form a bed when used in an aqueous system. Therefore, CEG materials by themselves are not considered suitable for use in the Arvia^{™} process. However, in view of the large surface area and low density, they may find use in a packed column of material through which contaminated gases could be passed.

### Reference Example 3 - Compressed Carbonised Impregnated Nyex^{™} (CCIN)

In order to overcome the difficulties posed by CEG materials, in particular the low density, a composite material of the CEG material and furfuryl alcohol derived carbon was investigated.

This material was produced by using CEG as a substrate on which carbon was grown. The carbon growth was in the form of pyrolysis of polymerised furfuryl alcohol, which has been impregnated onto the CEG. Energy-dispersive x-ray spectroscopy indicated a significant change to the surface of the material as a result of the impregnation.

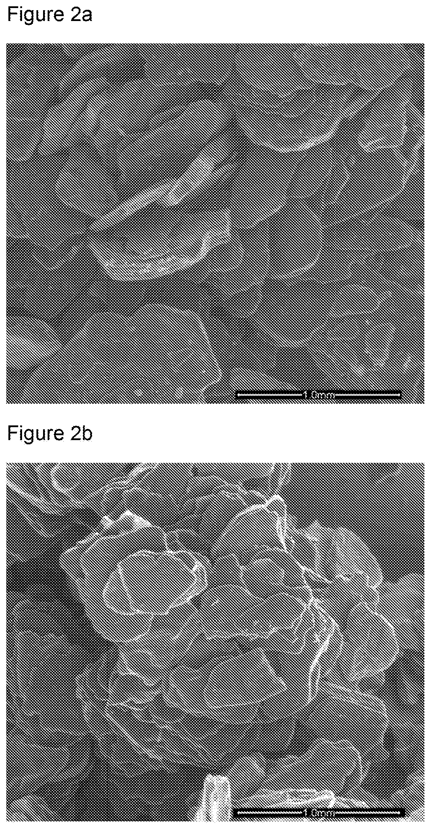

The NYEX ^{™} particles were sieved as described in Example 2 and then mixed with furfuryl alcohol. The furfuryl alcohol was polymerised and the resulting material was simultaneously carbonised and exfoliated since the elevated temperature at which carbonisation occurred was enough to exfoliate the NYEX^{™} particles. This was followed by compression with a force of 15,000 kg and size reduction. The compressed material was then crushed to form particles, which were measure to have an average particle size of 720 microns. Scanning electron microscope (SEM) images of Nyex^{™} and Nyex^{™} impregnated with furfuryl alcohol are shown in Figures 2a and 2b respectively.

A variety of CCIN materials were produced were developed by varying the mass ratio of furfuryl alcohol (FA) to CEG; the mass ratio of activating agent (KOH) and CEG; and the compression force. Details of the various materials are shown in Table 2

**Table 2**

| Material | Particle Density (g cm⁻³) | Compression Force (kg) | FA:CEG | KOH:CEG | Bulk Density (g cm⁻³) | Porosity |
|---|---|---|---|---|---|---|
| CCIN 1 | 0.796 | 10,000 | 3:2 | 3:1 | 0.22 | 0.724 |
| CCIN 2 | 1.094 | 10,000 | 2:1 | 3:1 | 0.27 | 0.753 |
| CCIN 3 | 1.161 | 10,000 | 2:1 | 1:1 | 0.29 | 0.750 |
| CCIN 4 | 1.200 | 15,000 | 2:1 | 1:1 | 0.39 | 0.675 |

The average surface area of the CCIN materials was measured by nitrogen adsorption as 24 m² g⁻¹, which is greater than the surface area of NYEX^{™}, but only marginally greater than the surface of the previous CEG material. The surface area of the furfuryl alcohol-derived carbon was measured as 0.254 m² g⁻¹, which suggests an insignificant contribution to the overall surface area of the material.

The densities of the various CCIN materials produced varied as shown in Table 2. The use of a higher proportion of furfuryl alcohol resulted in an increase composite density due to the formation of more furfuryl alcohol-derived carbon. Increasing the amount of KOH used during activation resulted in a lower density due to the increased porosity of the material. Further, extra compression forces increased the density due to increased compactness of the material.

The bed electrical conductivities of the CCIN composites were measured, and the results are shown in Table 3.

**Table 3**

| Current (mA) | Potential drop (V cm⁻¹) | Resistivity (Ω cm) | Conductivity (Ω⁻¹ cm⁻¹) |
|---|---|---|---|
| 50 | 0.053 | 6.53 | 0.15 |
| 100 | 0.106 | 6.53 | 0.15 |
| 200 | 0.209 | 6.43 | 0.16 |
| 400 | 0.417 | 6.42 | 0.16 |
| 800 | 0.826 | 6.36 | 0.16 |
| 1000 | 0.950 | 5.85 | 0.17 |

The average electrical conductivity was measured to be 0.16 ± 0.01 Ω⁻¹ cm⁻¹, which is comparable to NYEX^{™}. This suggests that these CCIN composites would be able to undergo electrochemical regeneration.

The adsorption kinetics of the CCIN materials for removal of AV-17 dye was investigated. Whilst the CCIN particles have increased surface area available for adsorption, the kinetics were found to be slower. Without wishing to be bound by scientific theory, it is believed that treating NYEX^{™} to make some of the internal surface available for adsorption increased the porosity of the material. Consequently, adsorption was no longer limited to the external surfaces and the intra diffusion part of the adsorption process becomes the rate-determining step. The amount of AV-17 removed from solution was greater than a comparative sample of NYEX^{™}.

Adsorption isotherms for the removal of AV-17 dye and resorcinol by the CCIN composites indicated that the CCIN materials adsorbed 6 mg g⁻¹ and 18 mg g⁻¹ for AV-17 and resorcinol respectively. This is around double the adsorption capacity for NYEX^{™}, which was measured at 3.5 mg g⁻¹ and 6 mg g⁻¹ for AV-17 and resorcinol respectively. Without wishing to be bound by scientific theory, it is believed that the larger size of the AV-17 dye molecules occupy a greater proportion of the surface of the adsorbent than the smaller resorcinol molecules.

A comparison of the regeneration efficiencies of the CCIN materials and NYEX^{™} is shown in Table 4.

**Table 4**

| | Regeneration Efficiency (%) | | | | |
|---|---|---|---|---|---|
| Charge Density (C g⁻¹) | Nyex | CCIN 1 | CCIN 2 | CCIN 3 | CCIN 4 |
| 8.6 | 100.2 | 63.5 | 64.2 | 65.8 | 65.7 |
| 12.9 | 101.2 | 76.1 | 75.8 | 76.4 | 76.8 |
| 17.1 | 100.6 | 87.3 | 88.1 | 88.6 | 88.6 |
| 21.4 | 101.4 | 100.1 | 100.2 | 100.7 | 100.4 |

Although the CCIN material showed comparable regeneration efficiencies as NYEX^{™}, they came at significantly increased energy costs as the low density nature of the CCIN particles resulted in less compact bed formation and consequently high electrical resistance across the bed.

At the same current densities as used with NYEX^{™}, the regeneration efficiencies of the CCIN materials were measured at around 63%, which is significantly lower than the efficiencies seen with NYEX^{™}, although the charge per unit pollutant removed was lower. Therefore, although the CCIN adsorbed between two and three times the amount of organics adsorbed by NYEX^{™}, the regeneration efficiency of 63% represents a lower charge per unit pollutant removed when compared to NYEX's 100% regeneration at 8.6 C g⁻¹ of adsorbent.

### Reference Example 4 - CEG and Granular Activated Carbon (GAC) Composite

The composite of CEG and carbon (CCIN) resulted in a material of low density and an uneven growth of carbon on the CEG matrix. As such, a composite of CEG and similarly sized granular activated carbon (GAC) using furfuryl alcohol as an impregnating material to bind the CEG and GAC particles together was produced.

NYEX^{™} was exfoliated as described previously to obtain expanded graphite which was compressed into CEG. The CEG was size reduced by crushing and mixed with GAC. The mixture of CEG and GAC was impregnated with furfuryl alcohol followed by polymerisation, as described previously. In particular, a mixture of 50 g of CEG and 50 g of granular activated carbon was impregnated with 100 grams of furfuryl alcohol, and then polymerised with HCl and heat. The material was then carbonised and activated as described previously.

The surface area of the resulting material was measured by nitrogen adsorption and analysed by BET surface area model as 16 m² g⁻¹. The density of the composite material was measured to be 1.39 kg m⁻³ by helium pycnometry, and the particles were measured to have an average particle diameter of 766 microns. The free particle settling velocity and the hindered settling velocity were estimated to be 159 cm min⁻¹ and 48 cm min⁻¹ respectively.

The dry bed electrical conductivity of the CEG-GAC composite material was measured to be 0.08 ± 0.01 Ω⁻¹ cm⁻¹. This is significantly lower than the conductivity of NYEX^{™}.

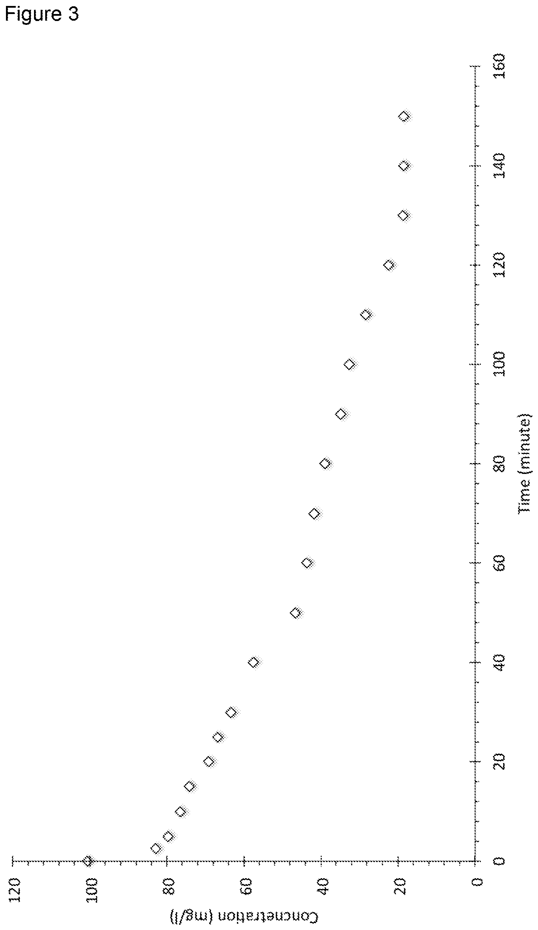

As shown in Figure 3, kinetics experiments with AV-17 showed that this composite material reached adsorption equilibrium after 140 minutes, which is slower than the other materials tested and suggests greater porosity of the material.

The adsorption isotherm of the CEG-GAC composite material showed a maximum loading of 16 mg g⁻¹, which is around a four and a half times increase of the capacity of NYEX^{™}. This is a significant improvement in adsorption capacity and represents excellent performance as an adsorbent, despite the longer adsorption equilibrium time.

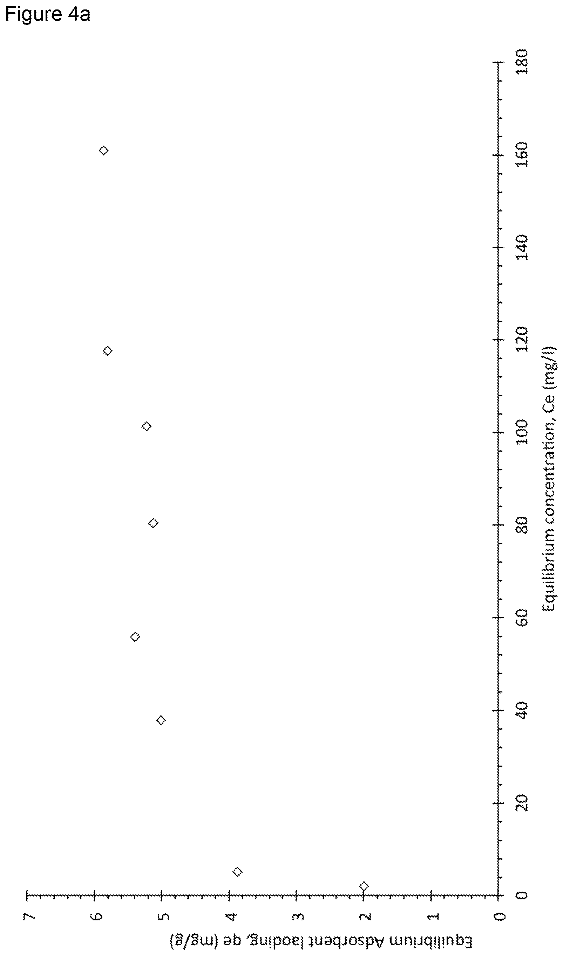

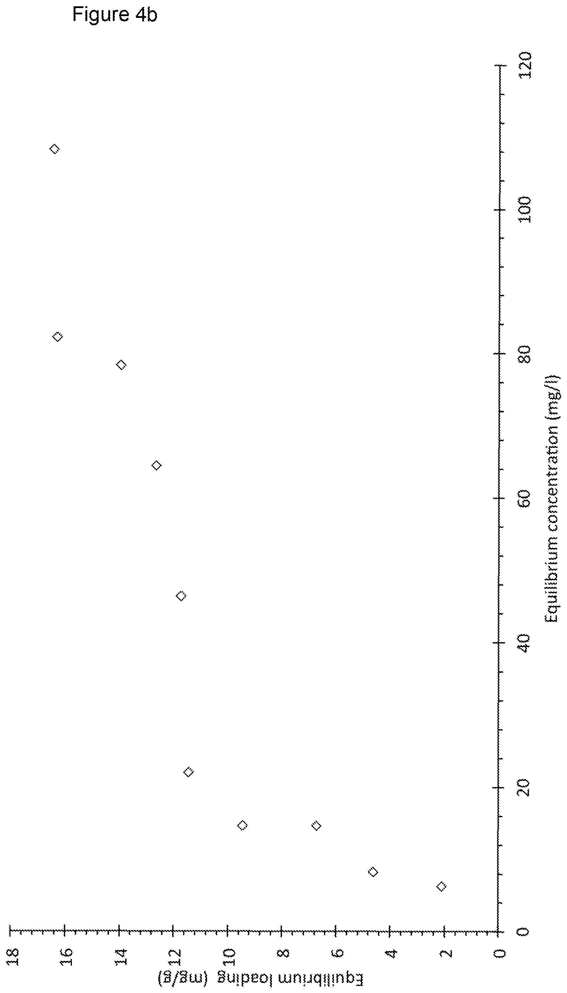

Two forms of the adsorbent were investigated: a non-activated one and an activated version. The performance of these two versions as adsorbents was tested using AV17 as the model pollutant. Figures 4a and 4b show the results of the adsorption experiments relating to these materials. In particular, Figure 4a shows the isotherm of adsorption of AV-17 onto non-activated CEG-GAC composite adsorbent and Figure 4b shows the isotherm of adsorption of AV-17 onto activated CEG-GAC composite adsorbent. The activated material adsorbed about three times as much as the non-activated material. These demonstrate the importance of activation in increasing the adsorption capacity of the adsorbent materials.

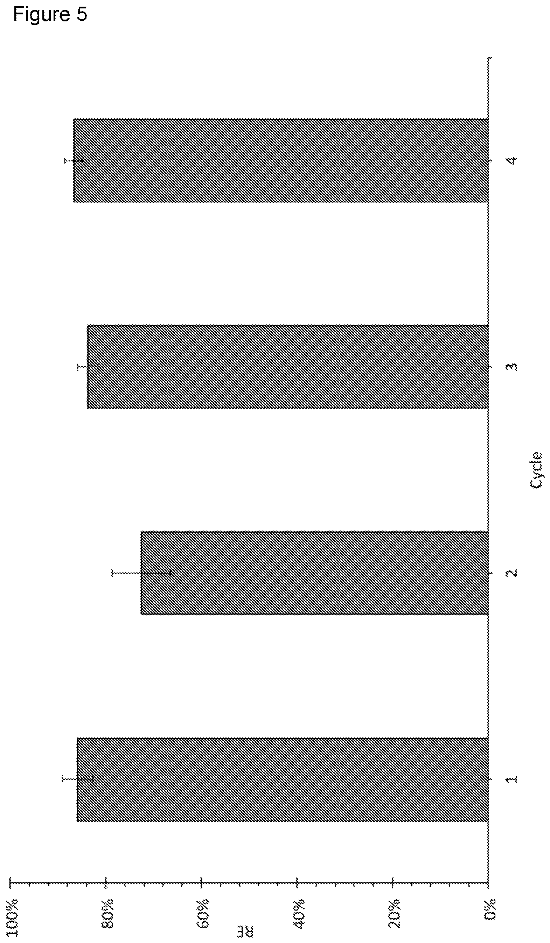

Regeneration efficiency experiments were carried out on the CEG-GAC composite materials. Four regeneration cycles were carried out and the regeneration efficiencies were significantly less than NYEX^{™}. Figure 5 shows the regeneration efficiencies of the CEG-GAC composite materials for the adsorption of AV-17 over four adsorption-regeneration cycles. Lower bed conductivities generally lead to higher cost of regeneration. Lower regeneration efficiencies may be due to a lower charge per unit mass of pollutant adsorbed.

Materials having different ratios of GAC and CEG were produced, and the GAC was replaced with powdered activated carbon, but without significant improvements.

### Reference Example 5 - Graphite and Activated Carbon

Carbonised Natural Flake Graphite was produced by using Natural Large Flake Graphite (NLFG) as a substrate upon which activated carbon derived from polymerised furfuryl alcohol was grown. The method was similar to that used to produce CCIN, with the NLFG replacing the NYEX^{™}. In particular, the NLFG was sieved and then impregnated with furfuryl alcohol. The furfuryl alcohol was then polymerised and the material was subsequently carbonised. The carbonised material was activated using KOH and then crushed. The NLFG activated carbon composite was washed by rinsing with distilled water until the pH was between 6 and 7.

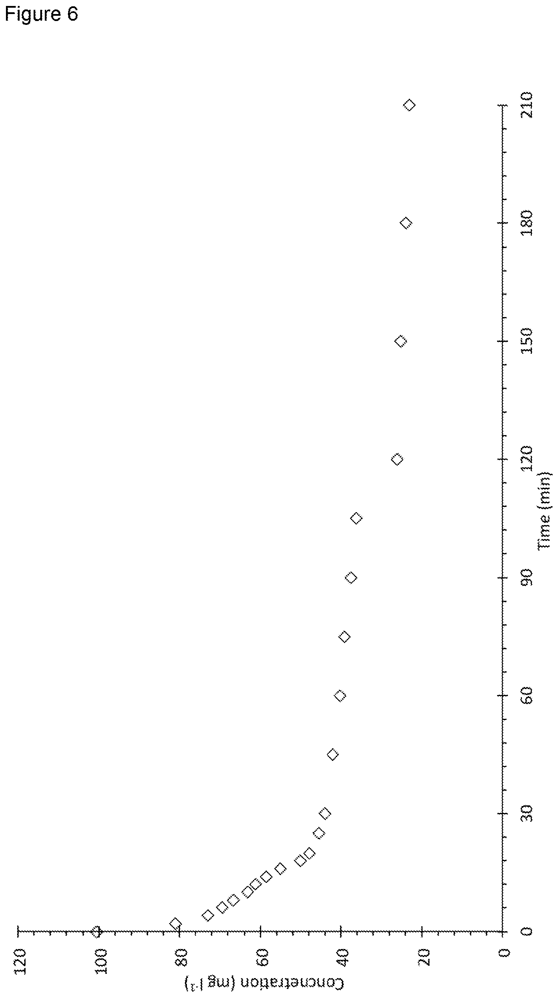

During kinetics experiments, this material was observed to undergo attrition and so the testing of this material was limited to the adsorption kinetics. Figure 6 shows a graph of the adsorption of AV-17 from solution by this composite adsorbent. The results showed that this material reached adsorption equilibrium after 210 minutes, which is characteristic of a porous material.

Following on from the composite formed by forming activated carbon by pyrolysis of polyfurfuryl alcohol on a graphite flake substrate, an alternative composite material was produced. In the alternative material, activated carbon particles were bound to the graphite flakes.

This material was produced by mixing NLFG with GAC, impregnating the mixture with furfuryl alcohol, polymerising the furfuryl alcohol, carbonising the resulting mixture, activating the composite material with KOH, crushing the activated composite, and washing the activated composite. Upon crushing, the material was observed to disintegrate. Increased ratios of furfuryl alcohol were used in further composites to try to increase the binding strength, but no significant increase in composite strength was observed.

In order to increase the binding strength, the GAC was replaced with powdered activated carbon. However, when the resulting material was characterised and tested, it was found that the coverage of the flake graphite particles by the powdered activated carbon was insufficient and irregular, and contributed to the low stability of the material.

### Example 6 - Powdered Graphite (PG) with Powdered Activated Carbon (PAC)

This example relates to a method in accordance with the first aspect of the present invention, as well as materials according to the second aspect of the present invention.

Previous attempts to produce a composite material of PG and PAC have not been successful. However, the methods of the first and fourth aspects of the present invention allow a composite of PG and PAC to be produced. The PG and PAC are mixed together and then impregnated/coated with furfuryl alcohol. The furfuryl alcohol is then polymerised and the mixture is subsequently carbonised. THE PG-PAC composite is then crushed and optionally washed.

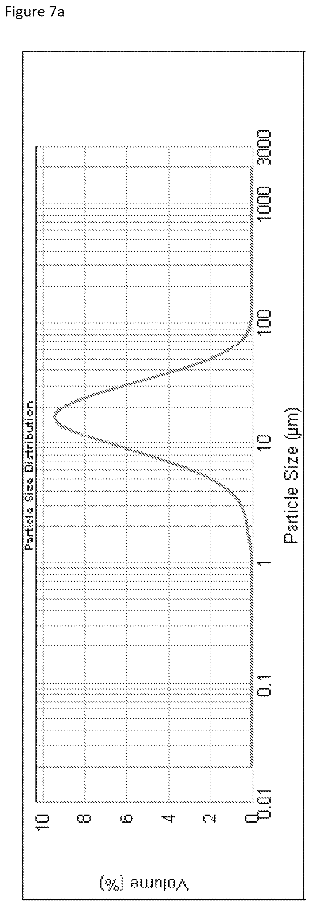

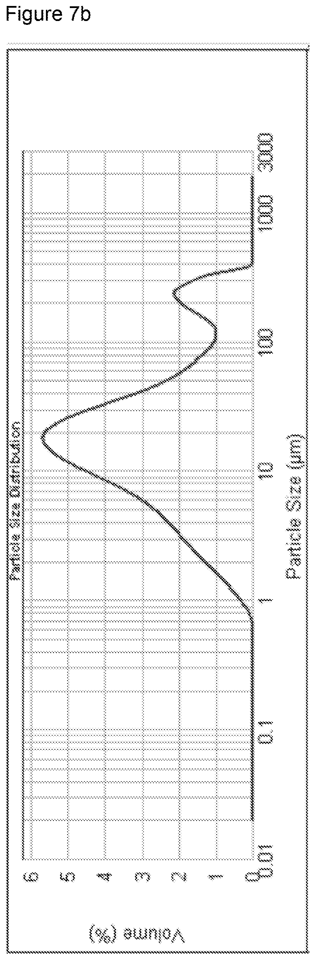

The carbonisation is undertaken under inert conditions, for example in a stream of nitrogen gas. The mean particle sizes of the PG and PAC are similar to enhance bonding. The PG and PAC were analysed on a Malvern DLS Mastersizer to measure their mean particle size distribution, and the results of these tests are shown in Figures 7a and 7b. In particular, Figure 7a shows the dynamic light scattering measurement showing the particle size distribution of powdered graphite and Figure 7b shows the particle size distribution of powdered activated carbon. The PG was measured to have a mean particle size of 16 microns and the PAC was measured to have a mean particle size of 18 microns. The surface areas of the powdered graphite and the powdered activated carbon were measure by BET nitrogen adsorption as 0.7 m² g⁻¹ and 1270 m² g⁻¹ respectively.

Three versions of the PG-PAC composite were developed. In the first version, an equal mass of PG and PAC were combined and will be referred to as PGPAC5050. Two other versions were also produced in which the PG and PAC were combined in ratios of 40:60 by mass and 60:40 by mass and will be referred to as PGPAC4060 and PGPAC6040.

The PGPAC4060 was developed with a view to increasing absorption capacity, and PGPAC6040 was developed with a view to increasing the regeneration ability.

Table 5 provides a summary of the surface areas, porosities, and settling velocities of the three PGPAC adsorbents.

**Table 5**

| | PGPAC4060 | PGPAC5050 | PGPAC6040 |
|---|---|---|---|
| BET Surface Area (m² g⁻¹) | 116.8 ± 5.1 | 24.3 ± 0.9 | 19.1 ± 1.3 |
| Micropore surface area (m² g⁻¹) | 101.9 | 18.2 | 15.9 |
| External surface area (m² g⁻¹) | 14.8 | 6.3 | 3.2 |
| Micropore contribution to total surface area (%) | 87.2 | 74.9 | 83.2 |
| Adsorbent bed porosity | 63.5 | 76.1 | 87.3 |
| Density (kg m⁻³) | 1550 | 1680 | 1805 |
| Particle settlement velocity (cm min⁻¹) | 205.0 | 238.9 | 269.1 |
| Hindered settlement velocity (cm min⁻¹) | 63.6 | 75.5 | 86.2 |

The effect of the ratio of the constituents had a large effect on the surface area, and therefore adsorptive capacities, of the composite materials. In particular, the composite with the greater ratio of PG to PAC had greater densities and settling velocities, although the differences in these properties were less significant than the difference in surface areas. As such, the gain of surface area shows a greater proportional increase than the gains in density and settling velocity.

These materials were measured to contain micropores and mesopores. The contributions of micropore surface areas and external surface areas are shown in Table 5, which shows that the PGPAC composite materials have high proportions of their surfaces as being micropores. These materials may therefore be classified as mainly microporous with between 17 and 25% of the surface area contributed by mesopores.

The electrical conductivity of a bed of each of the PGPAC variants was measured. The results show a positive correlation between the amount of powdered graphite in the composite and the electrical conductivity. PGPAC4060, which has the lowest amount of powdered graphite, was measured to have a bed conductivity of 0.28 ± 0.02 Ω⁻¹ cm⁻¹; PGPAC5050 was measured to have a bed conductivity of 0.32 ± 0.01 Ω⁻¹ cm⁻¹ and PGPAC6040 was measured to have a bed conductivity of 1.85 ± 0.05 Ω⁻¹ cm⁻¹.

This example demonstrates how the methods of the present invention can be used to produce tailored adsorbents. For example, where an adsorbent with a high adsorption capacity is required, the method can be used to produce an adsorbent with a high surface area, such as PGPAC4060, whereas if an adsorbent with good electrical conductivity is required, the method can be used to produce an adsorbent with high conductivity, such as PGPAC6040.

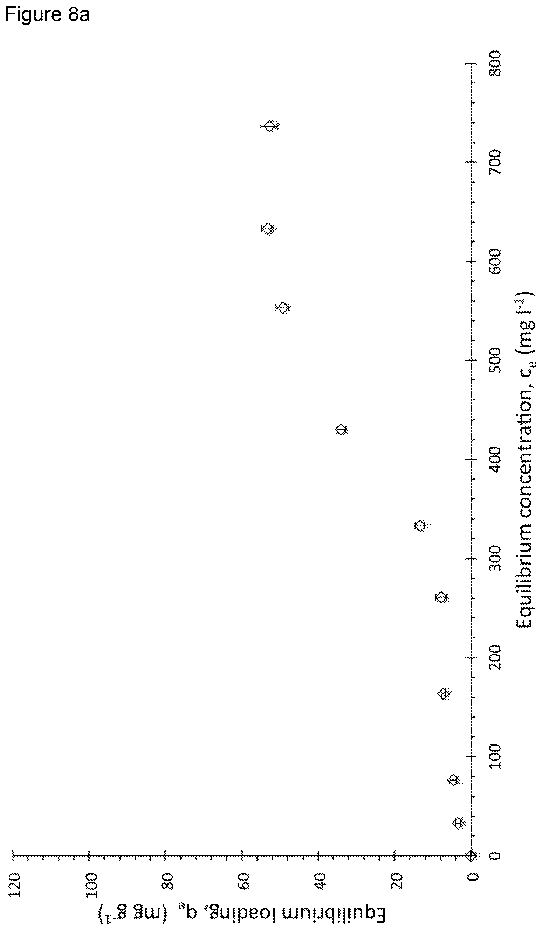

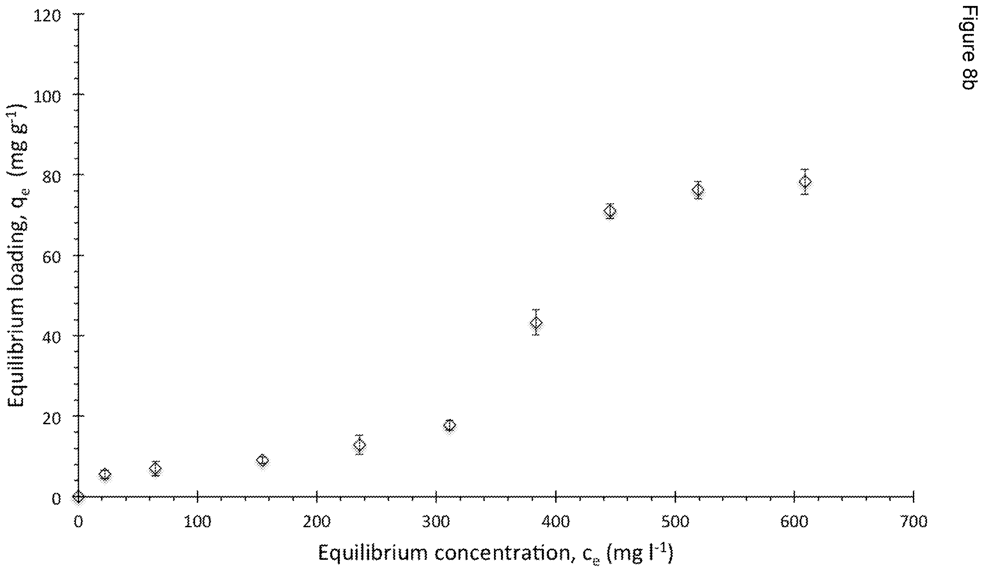

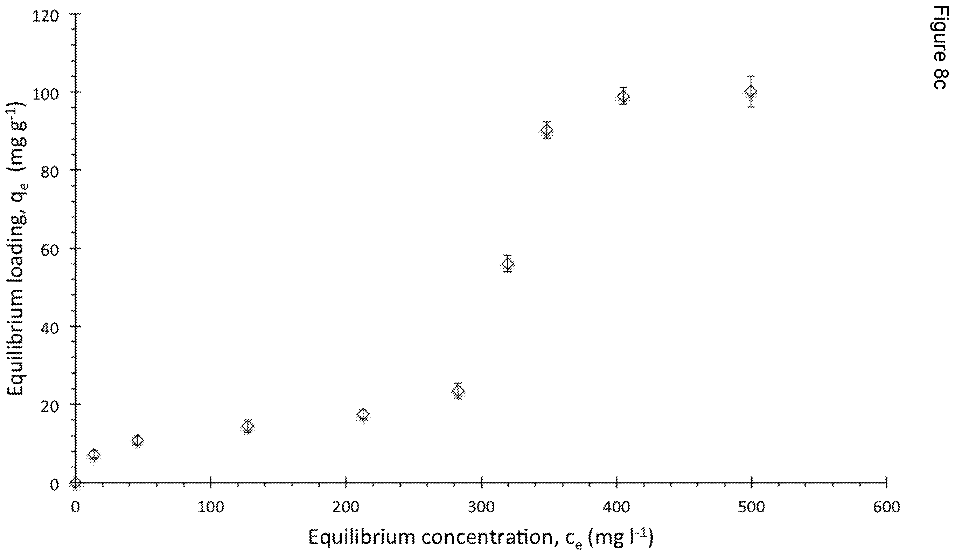

The adsorption kinetics of the three PGPAC variants were investigated. 50 g of the PGPAC variants was mixed into one litre solutions of AV-17 and resorcinol at concentrations of 100 ppm and 1000 ppm respectively, and the uptake was measured regularly. The kinetic results of the uptake of resorcinol by PGPAC6040, PGPAC5050, and PGPAC4060 are shown in Figures 8a, 8b, and 8c respectively. It was found that adsorption onto these PGPAC composites was slower than NYEX^{™}, which reached equilibrium after around 45 minutes. The PGPAC composite materials had not reached equilibrium after 300 minutes of adsorption. It is believed that this is a result of the larger surface area available for adsorption as well as the increased porosity of the PGPAC composite materials.

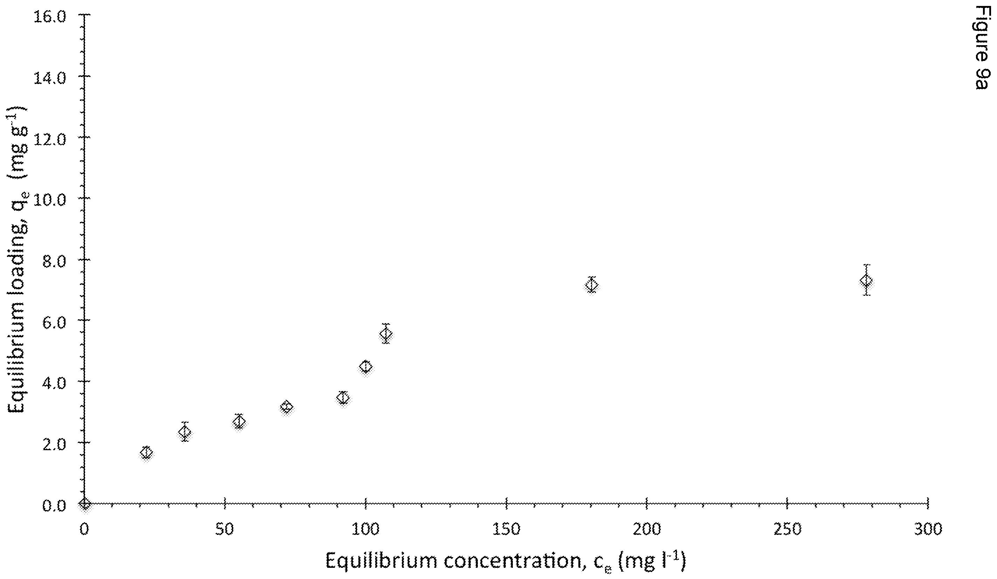

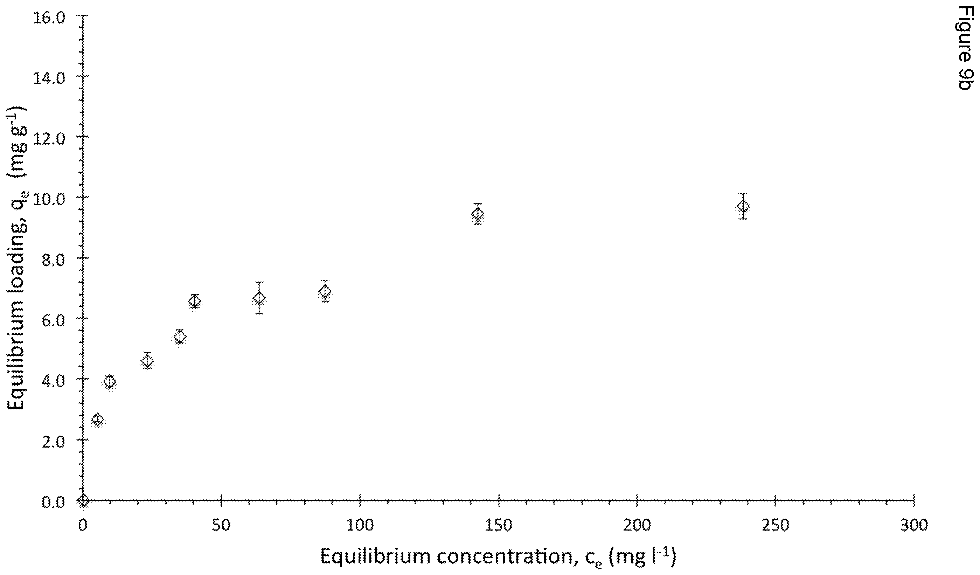

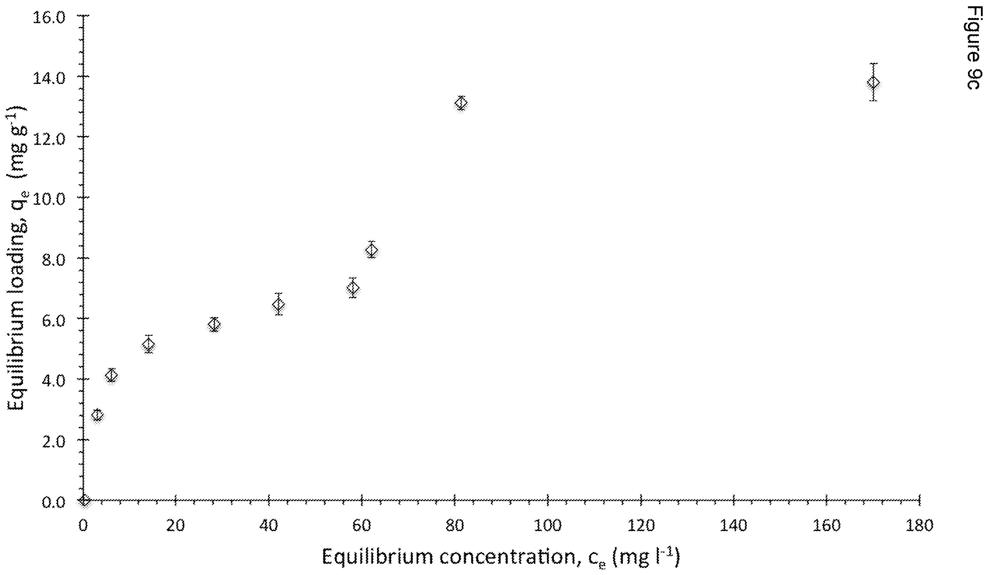

As shown in Figures 9a, 9b, and 9c, the uptake of AV-17 was similar to that of resorcinol by PGPAC6040, PGPAC5050, and PGPAC4060 respectively.

Although the PGPAC materials have longer adsorption equilibrium times, the rate of uptake of the resorcinol and AV-17 was significantly higher than the rate for NYEX^{™}. Figures 8a to c and 9a to c show that the PGPAC composites have significantly greater uptake rates than NYEX^{™}. In particular, after 60 minutes each of the PGPAC variants had adsorbed considerably more adsorbate than NYEX^{™}. As such, it does not appear necessary to wait until adsorption equilibrium has been reached to regenerate the adsorbent material.

The adsorption capacities for AV-17 were measured as 3.5 mg g⁻¹, 7.3 mg g⁻¹, 9.7 mg g⁻¹, and 13.8 mg g⁻¹ for NYEX^{™}, PGPAC6040, PGPAC5050, and PGPAC4060 respectively. The adsorption capacities for resorcinol were measured as 8.6 mg g⁻¹, 52.8 mg g⁻¹, 78.3 mg g⁻¹, and 100.1 mg g⁻¹ for NYEX^{™}, PGPAC6040, PGPAC5050, and PGPAC4060 respectively.

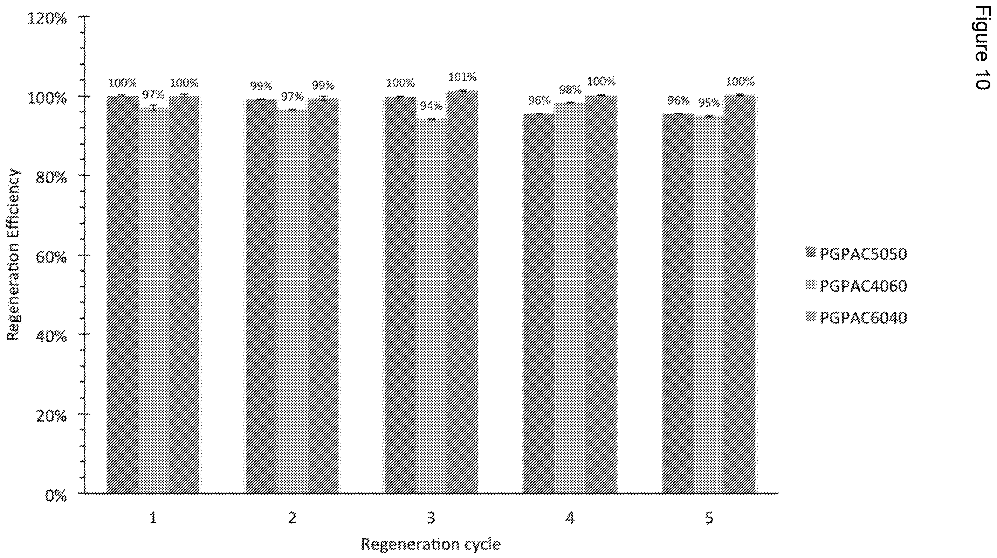

NYEX^{™} has been measured to have a sustained regeneration efficiency of over 100% over 5 cycles. The adsorption capacity of the NYEX^{™} material appears to improve after use, which is reflected in the efficiency in excess of 100%. Without wishing to be bound by scientific theory, it is believed that the passage of current through the adsorbent alters the surface properties of the adsorbent which increases its absorptive capacity. Figure 10 shows the adsorption efficiencies of the PGPAC variants over five cycles. This shows that surface modification of the NYEX^{™} can be achieved in situ as during treatment the contaminant surface coverage reduces, resulting in the modification of the surface enhancing adsorption. The averages regeneration efficiencies of the PGPAC were calculated to be 97.5%, 95%, and 100% for PGPAC5050, PGPAC4060, and PGPAC6040 respectively. The left hand bar in each group of three bars relates to PGPAC5050, the centre bar relates to PGPAC4060, and the right hand bar relates to PGPAC6040.

The stability of the PGPAC composite material variants was investigated since it has been observed that NYEX^{™} may undergo attrition over prolonged use.

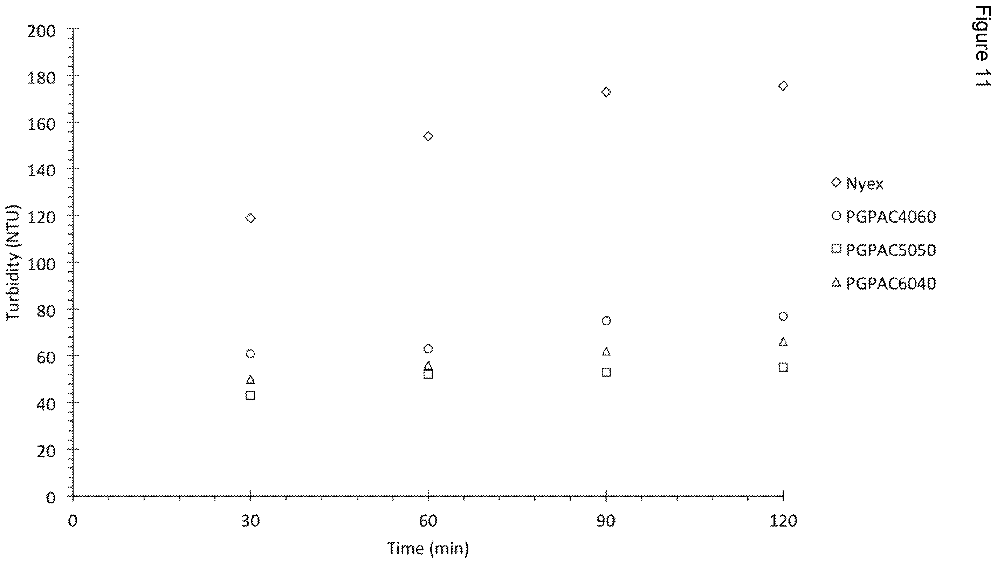

Figure 11 shows the results of bench scale isotherm experiments carried out in 500 ml conical flasks. A 4 g sample of NYEX^{™} was mixed with 100 ml of water and stirred continuously at 600 rpm using an ER Lauda magnetic stirrer. Samples of the water were taken every 30 minutes to be tested for turbidity. The samples were left to settle for ten minutes after being taken to allow the larger particles to settle, leaving the colloidal supernatant to be analysed for turbidity. Ten samples were taken at each measuring to allow for the calculation of a mean measurement.

The results indicate a steady increase in turbidity for the NYEX^{™} sample until 120 minutes after which only a marginal increase was noted. It is clear from Figure 11 that each of the PGPAC composites underwent significantly less attrition compared with NYEX^{™} particles.

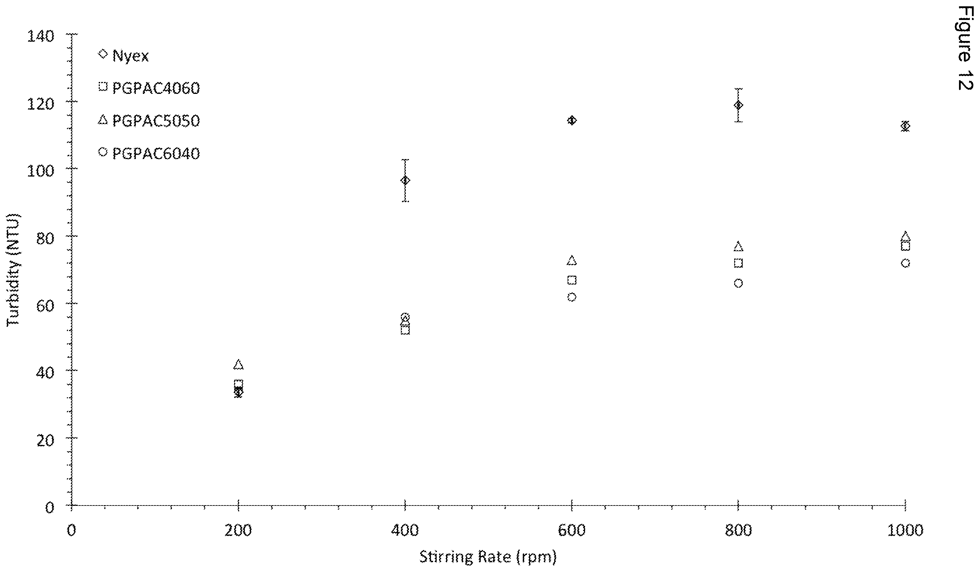

Further experiments to investigate the effect of the stirring rate on the attrition of the NYEX^{™} and PGPAC particles. These experiments revealed that the rate of attrition increased with stirring rate up to around 800 rpm after which it decreased slightly. Again, the rate of attrition of the NYEX^{™} particles was higher than that of the PGPAC particles. The results of these experiments are shown in Figure 12.

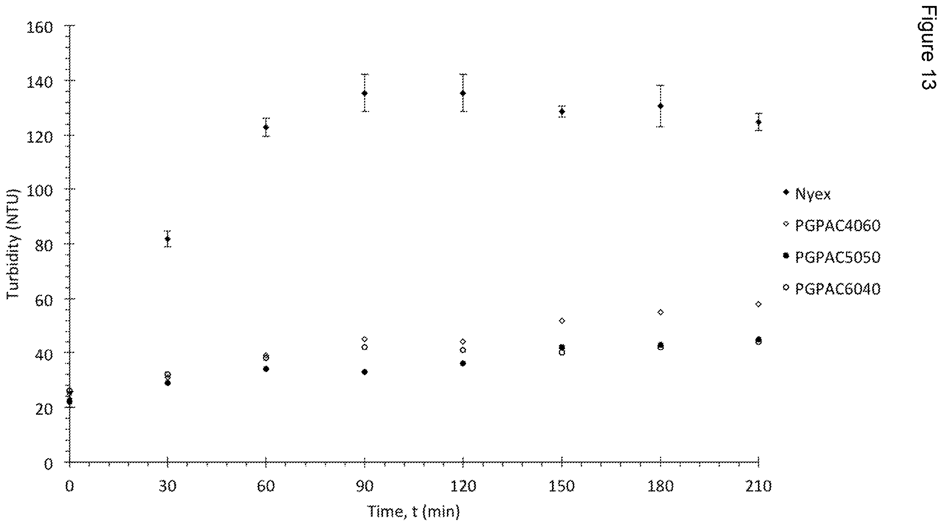

Still further experiments were carried out to investigate the effect of passing compressed air through the system. In one arrangement of the Arvia^{™} process, compressed air is passed through the bed of adsorbent and through the liquid to provide mixing and oxygen. In the present experiments, 100 g of adsorbent was mixed into 1 litre of water using compressed air at 2 barg at a flow rate of 2 litres per minute and samples were taken every 30 minutes for 210 minutes. As before, the samples were allowed to settle for ten minutes before the supernatant was collected and the turbidity measured. Figure 13 shows the results of these experiments. There was a significant increase in fines during the initial stage of mixing followed by saturation in the rate of formation of the fines. Again, the PGPAC materials showed improved resistance to attrition when compared with NYEX^{™}.

The effect of current passed through the adsorbent materials during electrochemical regeneration on the stabilities of the adsorbents was investigated by carrying out mock adsorption and regeneration without any adsorbates in the water.

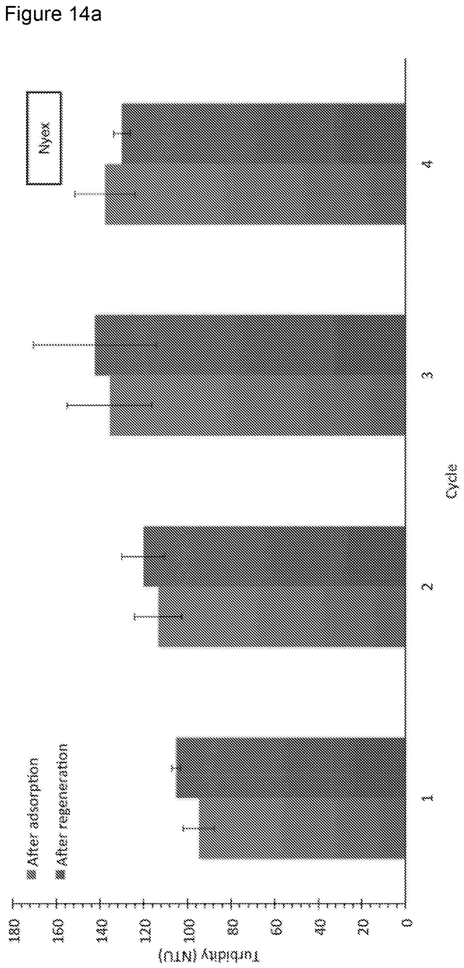

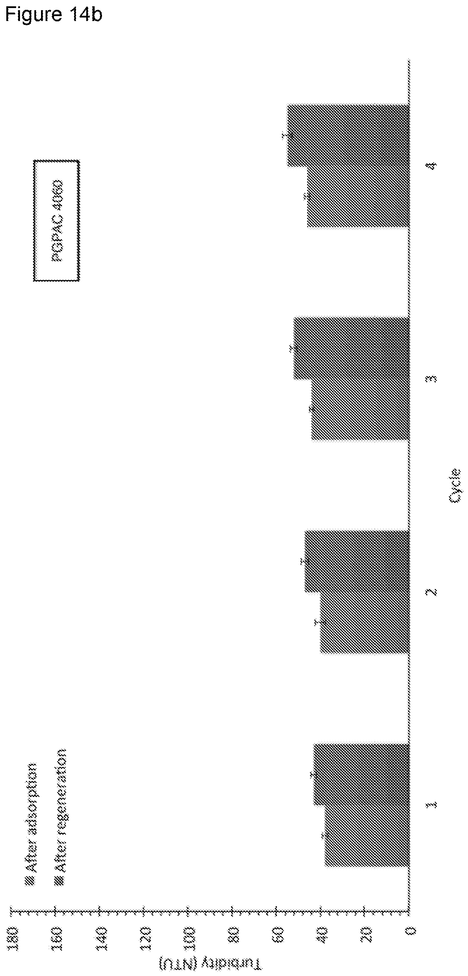

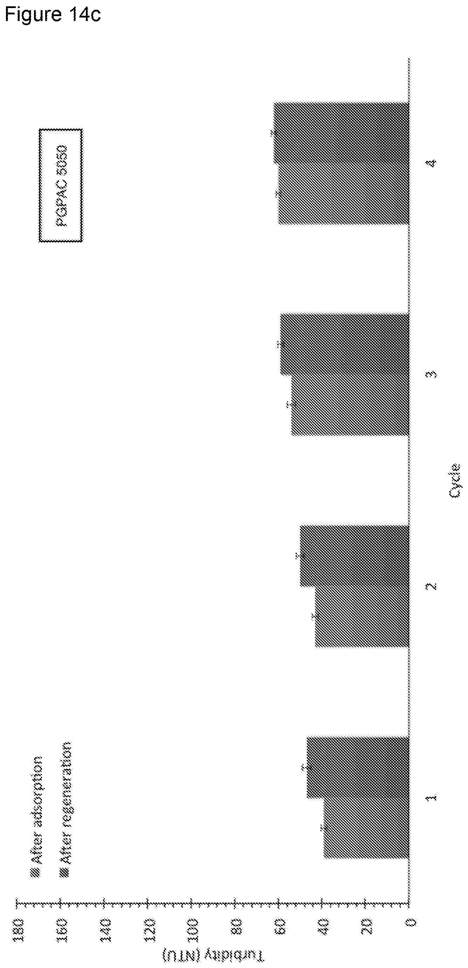

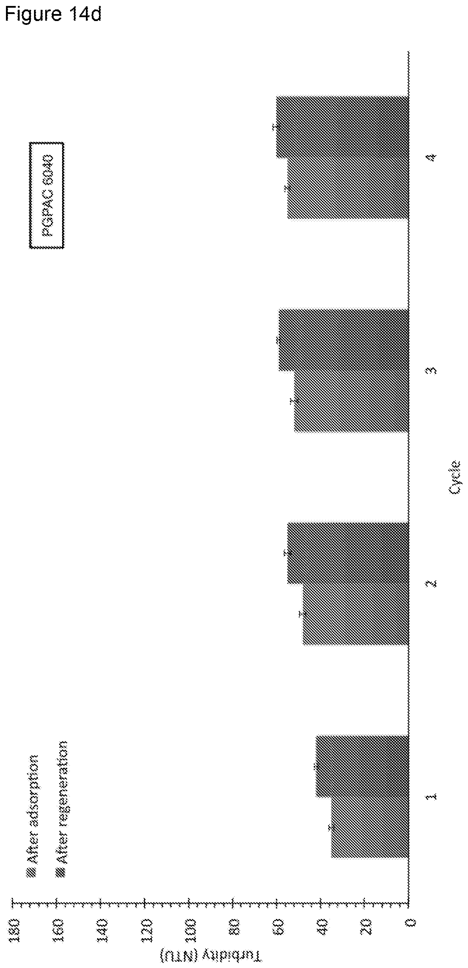

A test cell was filled with 100 g of adsorbent material and 1 litre of water. For the first mock adsorption, air was mixed into the system to replicate the mixing of the particles in water for 30 minutes. After 30 minutes, the air was stopped and the water was left to stand for 10 minutes to allow the solid adsorbent particles to settle in the regeneration zone. A mock regeneration was then carried out by passing a current of 1 A through the bed of adsorbent particles for 20 minutes at a charge density of 12 C g⁻¹. The active electrode area was 70 cm² and the cathode used was 0.3% acidified NaCl solution. The mock adsorption and regeneration were repeated over four cycles and the results are shown in Figures 14a, b, c, and d.

The results from the mock adsorption and regeneration experiments show a general trend which suggests that electrochemical treatment contributes to the attrition of the adsorbent. There was also a gradual increase in turbidity, which suggests that electrochemical regeneration increases attrition of the adsorbent.

In summary, the methods of the present invention allows for the production of adsorbent materials, the properties of which can be attuned to the particular environment in which the adsorbent materials are to be used. In particular, the methods may be used to produce an adsorbent material with a high adsorption capacity, or to produce an adsorbent material which has high conductivity. The adsorbent materials produced by the methods of the present invention may be treated in accordance with the fifth aspect of the present invention.

## Claims

1. A method of making a particulate adsorbent material for the treatment of contaminated liquids comprising mixing a first particulate material with a second material, wherein both the first and second materials are particulate, the first and second materials are different, the first material is electrically conductive, and the second material is adsorptive, homogenising the mixture of the first and second materials, incorporating an impregnating or coating material capable of carbonisation, and carbonising the mixture,
**characterized in that**:
the first material is powdered graphite and the second material is powdered activated carbon.

2. A method according to Claim 1, wherein the impregnating or coating material is curable, optionally wherein the mixture is cured prior to or simultaneously with carbonisation.

3. A method according to Claim 1 or Claim 2, wherein the first and second materials are mixed in any ratio, optionally wherein the first and second materials are mixed in ratios from around 1:99 to around 99:1, from around 10:90 to around 90:10, from around 20:80 to around 80:20, from around 30:70 to around 70:30, from around 40:60 to around 60:40, and around 50:50, all by weight.

4. A method according to any preceding claim, wherein the impregnating or coating material:
i. is one or a mixture of a thermosetting resin, a thermoplastic, or a monomer; and/or
ii. is a polymerisable organic compound.

5. A method according to Claim 4, wherein the impregnating or coating material is selected from phenolic resins, furan resins, oxidised polystyrene, polyvinyl alcohol, polyacrylonitrile, polyvinylidene chloride, cellulose, epoxy resins, polystyrene, sucrose, and polymethylmethacrylate; or is polyfurfuryl alcohol.

6. A method according to Claim 4 or Claim 5, wherein the impregnating or coating material is cured by thermal treatment, use of a chemical initiator, or a combination of the two, optionally wherein the chemical initiator is an acid or an alkali.

7. A method according to any preceding claim, wherein the material is activated, preferably following carbonisation, optionally wherein activation is effected by chemical and/or physical means, further optionally wherein:
i. the physical means comprises heating the material to a temperature sufficient to exfoliate the material and holding the material at that temperature for a time sufficient to allow exfoliation, optionally wherein the material is heated to around 800°C to around 900°C, further optionally wherein the material is purged with one or a combination of gases suitable for providing an oxidative atmosphere, yet further optionally wherein the gases are one or a combination of steam, carbon monoxide, and carbon dioxide; or
ii. the chemical means of activation is an acid, a salt, and/or a base, optionally wherein the chemical means is one or a combination of phosphoric acid, zinc chloride, potassium hydroxide, and sodium hydroxide; or
iii. activation comprises soaking the carbonised material in an aqueous solution of potassium hydroxide, optionally subjecting the material to vacuum-pressure cycles, drying the material, holding the material at a raised temperature to evaporate water, heating the material to around 700°C for a time sufficient to activate the material.

8. A method according to any preceding claim, wherein:
i. the mixture is dried before carbonisation, optionally wherein the material is crushed following carbonisation; and/or
ii. the material is washed, optionally wherein washing takes place after crushing; and/or
iii. the method includes subjecting the material to a reduced pressure for a period of time and subsequently allowing the pressure to return to atmospheric pressure, optionally wherein the vacuum-pressure cycle takes place before carbonisation.

9. A method according to Claim 1, wherein the impregnating or coating material is non-curable.

10. An adsorbent material which is made by, or is obtainable by, the method of any one of Claims 1 to 9, wherein the first material is powdered graphite, the second material is powdered activated carbon, and the impregnating or coating material is furfuryl alcohol.

11. The use of the adsorbent material of Claim 10 in the treatment of a contaminated liquid.

12. A method of treating the surface of an adsorbent material comprising passing a current through the adsorbent material in the absence of adsorbed contaminants, wherein the adsorbent material is made by, or obtainable by, the method of any one of Claims 1 to 9.

13. A method of treating the surface of an adsorbent material according to Claim 12, wherein the adsorptive capacity of the surface of the material is enhanced by electrochemical treatment, optionally wherein the enhancement is achieved in-situ prior to adsorption in the absence of an adsorbate and/or when an adsorbate is adsorbed to the surface of the adsorbent material.

14. A method of removing contaminants from a quantity of contaminated liquid comprising passing an electric current through an adsorbent material prior to contacting it with the contaminated liquid, contacting the adsorbent with the contaminated liquid, allowing the adsorbent material to adsorb contaminants from the contaminated liquid, and regenerating the adsorbent by passing an electric current through the adsorbent, wherein the adsorbent material is made by, or obtainable by, the method of any one of Claims 1 to 9.

## Patentansprüche

1. Verfahren zum Herstellen eines partikelförmigen adsorbierenden Material für die Behandlung von kontaminierten Flüssigkeiten, umfassend Mischen eines ersten partikelförmigen Materials mit einem zweiten Material, wobei sowohl das erste als auch das zweite Material partikelförmig sind, das erste und das zweite Material verschieden sind, das erste Material elektrisch leitend ist und das zweite Material adsorptiv ist, Homogenisieren der Mischung des ersten und des zweiten Materials, Inkorporieren eines Imprägnier- oder Beschichtungsmaterials, das zur Karbonisierung fähig ist, und Karbonisieren der Mischung,
**dadurch gekennzeichnet, dass**:
das erste Material pulverisierter Graphit ist und das zweite Material pulverisierte Aktivkohle ist.

2. Verfahren nach Anspruch 1, wobei das Imprägnier- oder Beschichtungsmaterial härtbar ist, wobei wahlweise die Mischung vor oder gleichzeitig mit der Karbonisierung gehärtet wird.

3. Verfahren nach Anspruch 1 oder Anspruch 2, wobei das erste und das zweite Material in einem beliebigen Verhältnis gemischt werden, wobei wahlweise das erste und das zweite Material in Verhältnissen von etwa 1:99 bis etwa 99:1, von etwa 10:90 bis etwa 90:10, von etwa 20:80 bis etwa 80:20, von etwa 30:70 bis etwa 70:30, von etwa 40:60 bis etwa 60:40 und etwa 50:50, alle nach Gewicht, gemischt werden.

4. Verfahren nach einem der vorhergehenden Ansprüche, wobei das Imprägnier- oder Beschichtungsmaterial:
i. eines von oder eine Mischung aus einem hitzehärtbaren Harz, einem Thermoplast oder einem Monomer ist und/oder
ii. eine polymerisierbare organische Verbindung ist.

5. Verfahren nach Anspruch 4, wobei das Imprägnier- oder Beschichtungsmaterial aus Phenolharzen, Furanharzen, oxidiertem Polystyrol, Polyvinylalkohol, Polyacrylnitril, Polyvinylidenchlorid, Cellulose, Epoxidharzen, Polystyrol, Saccharose und Polymethylmethacrylat ausgewählt ist; oder Polyfurfurylalkohol ist.

6. Verfahren nach Anspruch 4 oder Anspruch 5, wobei das Imprägnier- oder Beschichtungsmaterial durch thermische Behandlung, Verwendung eines chemischen Initiators oder einer Kombination der beiden gehärtet wird, wobei wahlweise der chemische Initiator eine Säure oder ein Alkali ist.

7. Verfahren nach einem der vorhergehenden Ansprüche, wobei das Material aktiviert wird, vorzugsweise nach der Karbonisierung, wobei wahlweise die Aktivierung durch chemische und/oder physikalische Mittel bewirkt wird, ferner wobei wahlweise:
i. das physikalische Mittel das Erhitzen des Materials auf eine Temperatur, die ausreicht, um das Material zu exfolieren, und das Halten des Materials bei dieser Temperatur für eine Zeit, die ausreicht, um Exfoliation zu gestatten, umfasst, wobei wahlweise das Material auf etwa 800 °C bis etwa 900 °C erhitzt wird, ferner wobei wahlweise das Material mit einem oder einer Kombination von Gasen gespült wird, die geeignet sind, eine oxidative Atmosphäre bereitzustellen, wobei ferner die Gase wahlweise eine oder eine Kombination von Dampf, Kohlenmonoxid und Kohlendioxid sind; oder
ii. das chemische Mittel zur Aktivierung eine Säure, ein Salz und/oder eine Base ist, wobei wahlweise das chemische Mittel eine Kombination von Phosphorsäure, Zinkchlorid, Kaliumhydroxid und Natriumhydroxid ist; oder
iii. die Aktivierung das Einweichen des karbonisierten Materials in einer wässerigen Lösung von Kaliumhydroxid, wahlweise das Aussetzen des Materials Vakuumdruckzyklen, das Trocknen des Materials, das Halten des Materials bei einer erhöhten Temperatur, um Wasser zu verdampfen, das Erhitzen des Materials auf etwa 700 °C für eine Zeit, die ausreicht, das Material zu aktivieren, umfasst.

8. Verfahren nach einem der vorhergehenden Ansprüche, wobei:
i. die Mischung vor der Karbonisierung getrocknet wird, wobei wahlweise das Material nach der Karbonisierung zerkleinert wird; und/oder
ii. das Material gewaschen wird, wobei wahlweise das Waschen nach der Zerkleinerung erfolgt; und/oder
iii. das Verfahren Folgendes einschließt: das Material einem reduzierten Druck für eine Zeitperiode auszusetzen und anschließend dem Druck zu gestatten, zum atmosphärischen Druck zurückzukehren, wobei wahlweise die Vakuumdruckzyklen vor der Karbonisierung stattfinden.

9. Verfahren nach Anspruch 1, wobei das Imprägnier- oder Beschichtungsmaterial nicht härtbar ist.

10. Adsorbierendes Material, das durch das Verfahren nach einem der Ansprüche 1 bis 9 hergestellt wird oder erhältlich ist,
wobei das erste Material pulverisierter Graphit ist, das zweite Material pulverisierte Aktivkohle ist und das Imprägnier- oder Beschichtungsmaterial Furfurylalkohol ist.

11. Verwendung des adsorbierenden Materials nach Anspruch 10 bei der Behandlung einer kontaminierten Flüssigkeit.

12. Verfahren zur Behandlung der Oberfläche eines adsorbierenden Materials, Folgendes umfassend: einen Strom durch das adsorbierende Material bei Abwesenheit adsorbierender Kontaminationen zu leiten, wobei das adsorbierende Material durch das Verfahren nach einem der Ansprüche 1 bis 9 hergestellt wird oder erhältlich ist.

13. Verfahren zur Behandlung der Oberfläche eines adsorbierenden Materials nach Anspruch 12, wobei die Adsorptionskapazität der Oberfläche des Materials durch eine elektrochemische Behandlung verbessert wird, wobei wahlweise die Verbesserung in situ vor der Adsorption bei Abwesenheit eines Adsorbats und/oder, wenn ein Adsorbat an der Oberfläche des adsorbierenden Materials adsorbiert wird, erreicht wird.

14. Verfahren zum Entfernen von Kontaminationen aus einer Menge einer kontaminierten Flüssigkeit, Folgendes umfassend: einen elektrischen Strom durch ein adsorbierendes Material zu leiten, bevor es mit der kontaminierten Flüssigkeit in Kontakt gebracht wird, das Adsorbens mit der kontaminierten Flüssigkeit in Kontakt zu bringen, zu gestatten, dass das adsorbierende Material Kontaminationen aus der kontaminierten Flüssigkeit adsorbiert, und das Adsorbens zu regenerieren, indem ein elektrischer Strom durch das Adsorbens geleitet wird, wobei das adsorbierende Material durch das Verfahren nach einem der Ansprüche 1 bis 9 hergestellt wird oder erhältlich ist.

## Revendications

1. Procédé de fabrication d'un matériau adsorbant particulaire pour le traitement de liquides contaminés, comprenant le mélange d'un premier matériau particulaire avec un deuxième matériau, dans lequel les premier et les deuxième matériaux sont tous deux particulaires, les premier et deuxième matériaux sont différents, le premier matériau est électriquement conducteur, et le deuxième matériau est adsorbant, l'homogénéisation du mélange du premier et du deuxième matériau, l'incorporation d'un matériau d'imprégnation ou de revêtement apte à une carbonisation, et la carbonisation du mélange,
**caractérisé en ce que** :
le premier matériau est du graphite en poudre et le deuxième matériau est du charbon actif en poudre.

2. Procédé selon la revendication 1, dans lequel le matériau d'imprégnation ou de revêtement est durcissable, optionnellement dans lequel le mélange est durci avant ou en même temps que la carbonisation.

3. Procédé selon la revendication 1 ou 2, dans lequel le premier et le deuxième matériau sont mélangés dans un rapport quelconque, optionnellement dans lequel le premier et le deuxième matériau sont mélangés dans des rapports d'environ 1:99 à environ 99:1, d'environ 10:90 à environ 90:10, d'environ 20:80 à environ 80:20, d'environ 30:70 à environ 70:30, d'environ 40:60 à environ 60:40, et d'environ 50:50, tous se rapportant au poids.

4. Procédé selon l'une quelconque des revendications précédentes, dans lequel le matériau d'imprégnation ou de revêtement :
i. est un ou un mélange des suivants : une résine thermodurcissable, un thermoplastique, ou un monomère, et/ou
ii. est un composé organique polymérisable.

5. Procédé selon la revendication 4, dans lequel le matériau d'imprégnation ou de revêtement est sélectionné parmi des résines phénoliques, des résines de furane, du polystyrène oxydé, de l'alcool de polyvinyle, du polyacrylonitrile, du polychlorure de vinylidène, de la cellulose, des résines époxy, du polystyrène, du sucrose et du poly(méthacrylate de méthyle), ou est un alcool polyfurfurylique.

6. Procédé selon la revendication 4 ou 5, dans lequel le matériau d'imprégnation ou de revêtement est durci par un traitement thermique, l'utilisation d'un amorceur chimique, ou une combinaison des deux, optionnellement dans lequel l'amorceur chimique est un acide ou un alcali.

7. Procédé selon l'une quelconque des revendications précédentes, dans lequel le matériau est activé, de préférence après une carbonisation, optionnellement dans lequel l'activation est réalisée par un moyen chimique et/ou physique, en outre dans lequel, optionnellement :
i. le moyen physique comprend le chauffage du matériau à une température suffisante pour exfolier le matériau et le maintien du matériau à cette température pendant une durée suffisante pour permettre l'exfoliation, optionnellement dans lequel le matériau est chauffé à une température d'environ 800 °C à environ 900 °C, optionnellement en outre dans lequel le matériau est purifié avec un gaz ou une combinaison de gaz appropriés pour fournir une atmosphère oxydante, et optionnellement en outre dans lequel les gaz sont un ou une combinaison des suivants : de la vapeur d'eau, du monoxyde de carbone et du dioxyde de carbone, ou
ii. le moyen chimique d'activation est un acide, un sel et/ou une base, optionnellement dans lequel le moyen chimique est un ou une combinaison des suivants : de l'acide phosphorique, du chlorure de zinc, de l'hydroxyde de potassium et de l'hydroxyde de sodium, ou
iii. l'activation comprend l'immersion du matériau carbonisé dans une solution aqueuse d'hydroxyde de potassium, optionnellement la soumission du matériau à des cycles de pression négative, le séchage du matériau, le maintien du matériau à une température élevée pour évaporer de l'eau, le chauffage du matériau à environ 700 °C pendant une durée suffisante pour activer le matériau.

8. Procédé selon l'une quelconque des revendications précédentes, dans lequel :
i. le mélange est séché avant une carbonisation, optionnellement dans lequel le matériau est broyé après la carbonisation, et/ou
ii. le matériau est lavé, optionnellement dans lequel le lavage s'effectue après le broyage, et/ou
iii. le procédé inclut la soumission du matériau à une pression réduite pendant une certaine période et ensuite le fait de laisser la pression revenir à la pression atmosphérique, optionnellement dans lequel le cycle de pression négative est réalisé avant la carbonisation.

9. Procédé selon la revendication 1, dans lequel le matériau d'imprégnation ou de revêtement est non durcissable.

10. Matériau adsorbant qui est fabriqué par, ou peut être obtenu par, le procédé selon l'une quelconque des revendications 1 à 9,
dans lequel le premier matériau est du graphite en poudre, le deuxième matériau est du charbon actif en poudre, et le matériau d'imprégnation ou de revêtement est de l'alcool furfurylique.

11. Utilisation du matériau adsorbant selon la revendication 10 dans le traitement d'un liquide contaminé.

12. Procédé de traitement de la surface d'un matériau adsorbant comprenant le fait de faire passer un courant à travers le matériau adsorbant en l'absence de contaminants adsorbés, dans lequel le matériau adsorbant est fabriqué par, ou peut être obtenu par le procédé selon l'une quelconque des revendications 1 à 9.

13. Procédé de traitement de la surface d'un matériau adsorbant selon la revendication 12, dans lequel la capacité d'adsorption de la surface du matériau est renforcée par un traitement électrochimique, optionnellement dans lequel le renforcement est réalisé in situ avant l'adsorption en l'absence d'un adsorbat et/ou lorsqu'un adsorbat est adsorbé sur la surface du matériau adsorbant.

14. Procédé pour éliminer des contaminants à partir d'une quantité de liquide contaminé, comprenant le fait de faire passer un courant électrique à travers un matériau adsorbant avant de le mettre en contact avec le liquide contaminé, la mise en contact du matériau adsorbant avec le liquide contaminé, le fait de laisser le matériau adsorbant adsorber des contaminants du liquide contaminé, et la régénération du matériau adsorbant en faisant passer un courant électrique à travers l'adsorbant, dans lequel le matériau adsorbant est fabriqué par, ou peut être obtenu par le procédé selon l'une quelconque des revendications 1 à 9.
